# EUROPEAN PATENT APPLICATION

(11) **EP 2 511 289 A1**
(43) Date of publication of application: **17.10.2012**
(21) Application number: 11003142.4
(22) Date of filing: 14.04.2011
(51) Int. Cl.: C07K 9/00, C07K 14/47, A61K 47/48

(54) **Method for the rapid chemoenzymatic glycosylation of conjugates between peptides and hydrophilic polymers**

(71) Applicant: Universität zu Köln, 50923 Köln (DE); Technische Universität München, 85747 Garching (DE)
(72) Inventor: Schwientek, Tilo, Dr., 41468 Neuss (DE); Becker, Christian Friedrich Wilhelm, Prof. Dr., 85748 Garching b. München (DE)
(74) Representative: Lahrtz, Fritz

(57) **Abstract**

The present invention relates to a method for the production of a glycosylated peptide comprising providing a peptide conjugated with one or more hydrophilic polymer(s) and glycosylating the polymer-conjugated peptide by means of one or more glycosyltransferase(s).

SEQUENCE LISTING

<110>  Universität zu Köln; Technische Universität
       München

<120>  Polymer-Conjugate Technology for the Rapid Chemoenzymatic
       Synthesis of Glycopeptides

<130>  P67630EP

<160>  4

<170>  PatentIn version 3.5

<210>  1
<211>  18
<212>  PRT
<213>  Homo sapiens

<400>  1

Ala His Gly Val Thr Ser Ala Pro Asp Thr Arg Pro Ala Pro Gly Ser
1               5                   10                  15

Thr Ala

<210>  2
<211>  26
<212>  PRT
<213>  Artificial Sequence

<220>
<223>  mucin 1-derived peptide elongated at the N-terminus by a TEV
       cleavage site

<400>  2

Gly Asp Glu Asn Leu Tyr Phe Gln Ala His Gly Val Thr Ser Ala Pro
1               5                   10                  15

Asp Thr Arg Pro Ala Pro Gly Ser Thr Ala
            20                  25

<210>  3
<211>  19
<212>  PRT
<213>  Artificial Sequence

<220>
<223>  mucin 1-derived peptide with N-terminal PEG27 moiety

<220>
<221>  X
<222>  (1)..(1)
<223>  HO-(polyethylene glycol)27

<220>
<221>  X
<222>  (1)..(1)
<223>  HO-(polyethylene glycol)27 moiety

<220>

<221>  X
<222>  (1)..(1)
<223>  HO-(polyethylene glycol)27 (PEG27)

<400>  3

Xaa Ala His Gly Val Thr Ser Ala Pro Asp Thr Arg Pro Ala Pro Gly
1               5                   10                  15

Ser Thr Ala

<210>  4
<211>  27
<212>  PRT
<213>  Artificial Sequence

<220>
<223>  mucin 1-derived ppeptide sequence elongated at the N-terminus by
       a TEV cleavage site with N-terminal PEG27 moiety

<220>
<221>  X
<222>  (1)..(1)
<223>  PEG27 moiety

<220>
<221>  X
<222>  (1)..(1)
<223>  HO-(polyethylene glycol)27 moiety

<400>  4

Xaa Gly Asp Glu Asn Leu Tyr Phe Gln Ala His Gly Val Thr Ser Ala
1               5                   10                  15

Pro Asp Thr Arg Pro Ala Pro Gly Ser Thr Ala
            20                  25

## Description

The present invention relates to a method for the production of a glycosylated peptide comprising providing a peptide conjugated with one or more hydrophilic polymer(s) and glycosylating the polymer-conjugated peptide by means of one or more glycosyltransferase(s).

In nature, glycosylation occurs as a highly abundant post-translational modification of peptides. Here, carbohydrate moieties are typically conjugated with amino groups (N-glycosylation), hydroxyl groups (O-glycosylation), or thiol groups (S-glycosylation) by enzymatic catalysis. Often, glycosylation involves highly complex enzymatic cascades comprising two, three, four or more different enzymes. Glycosylated peptides may also be designated glycopeptides.

Glycosylation is known to play a key role in numerous biological and biochemical functions such as, e.g., protein folding, cellular differentiation, cell-cell communication, cell-matrix interaction and viral invasion. Accordingly, glycosylation may play a crucial role in different types of biochemical recognition processes responsible for growth, development, infection, immune response, cell adhesion, signal transduction processes, formation and manifestation of neoplasia, cancer, metastases and autoimmune diseases. Moreover, the glycosylation pattern of a peptide may have a great impact on the immunogenicity and the proteolytic stability of said peptide. A glycopeptide itself and/or a carbohydrate moiety cleaved off said glycopeptide may serve as an epitope. Further, glycosylation may influence the sliminess of mucin proteins and mucin peptides occurring in mucosa.

Today, many glycopeptides are of great interest for pharmaceutical purposes. Herein, a glycopeptide may serve as, e.g., antibiotic, hormone and cytokine (e.g., juvenile human growth hormone, CD4 or tissue plasminogen activator), vaccine, artificial extracellular matrix, artificial glycocalyx or a coating for implants. Further, numerous glycopeptides are of great interest for research purposes.

So far, glycopeptides are mostly obtained from natural sources. However, purification of said peptides is highly challenging and laborious. Additionally, the glycopeptides found in nature are mostly proteins of more than 100 amino acids in length, often comprising more than one glycosylation site(s) and their glycosylation pattern cannot be fully controlled. Therefore, there is still the need for efficient methods for producing glycopeptides by synthetical means.

In general, the chemical synthesis of glycopeptides imposes high requirements with regard to the reversibility and selectivity of protecting groups and to the stereoselectivity of the formation of glycosidic bonds. Currently, there are two common strategies for the synthesis of glycopeptides known in the art. According to the first strategy, a peptide strand is synthesized (e.g., by solid phase peptide synthesis (SPPS) as well-known by those skilled in the art (Merrifield, 1963; Carpino and Han, 1972)) and, during the synthesis of said peptide, one or more glycosylated amino acid building block(s) are integrated in the sequence instead of standard amino acid building block(s) (Becker et al., 2006). According to the second strategy, a peptide is first fully synthesized (e.g., by SPPS), then, subsequently, one or more protecting groups are cleaved off selectively and the carbohydrate building block is conjugated to the peptide (Becker et al., 2006).

As known by those skilled in the art, both strategies bear several severe drawbacks. First, both strategies require the use of fully protected carbohydrate building blocks, which may cause problems when being deprotected. Protecting group strategies become highly complex as the carbohydrate moiety as well as the peptide requires different orthogonal protecting groups. As chemical methods are not fully stereospecific, the obtained peptides are not pure with respect to chirality. The second strategy, thus, the subsequent conjugation of a carbohydrate building block to a peptide, is further hampered by side reactions such as, e.g., aspartimide formation in the case of N-glycosides or difficulties in forming stereoselective O-glycosylation reactions with complex targets. Moreover, the provision of the required carbohydrate building blocks is highly challenging. Further, the yields of the glycosylated peptides are typically rather poor as several of the steps of coupling and deprotection are not quantitative. Further, purification has regularly to be performed in a multistep procedure that further decreases the obtainable yields. Finally, the coupling step of each carbohydrate moiety typically requires the extensive use of highly toxic agents such as, e.g, trifluoroacetic acid (TFA), mutagenic or allergenic coupling reagents, acetonitrile, dimethylformamide as solvent and scavenger agents. Even a standard purification method such as, e.g., reversed phase high performance liquid chromatography (RP-HPLC) is typically performed by using a toxic eluent comprising acetonitrile and TFA.

Therefore, there is the need for an alternative method.

Alternatively, in principle, glycosylated peptides should be obtainable from methods using glycosylating enzymes. However, it will be understood by a person skilled in the art that after each step of the glycosylation reaction that is completed, the glycosylating enzyme and the obtained glycopeptide should be separated from another. When there are more than one glycosylating steps, this is of particular importance. Then, the glycosylating enzyme, the glycosyltransferase, of the first glycosylating step must be removed by purifying the peptide and, subsequently, a second glycosylating enzyme has to be added and so forth. Typically, purification is performed by means of several chromatographic methods such as, e.g., RP-HPLC. However, separation of the obtained glycopeptides from the enzyme is often difficult and typically leads to a considerable loss of peptide yield, as a glycopeptide, in particular a hydrophobic glycopeptide, typically sticks to the column matrix. Additionally, as described above, RP-HPLC is typically performed by using toxic eluents comprising acetonitrile and TFA, as the peptide may not be soluble in pure water or an aqueous standard buffer. As acetonitrile and TFA residuals are known to disturb the successive glycosyltransferase reaction, these components have to be extensively removed. Removing the components of the preceding glycosylating step is typically a multistep procedure involving HPLC, identification and pooling of positive fractions, excessive drying and reconstitution of the glycopeptide in appropriate buffers. The person skilled in the art will notice that each purification step will decrease the yield of the glycosylated peptide. In particular when more than one saccharide units are conjugated to one peptide, thus, when there are two or more glycosylating step(s), each glycosylating step requires subsequent separation steps and, consequently, leads to a decrease of peptide yield, the enzyme-based method will evidently lose its attractiveness as the yield becomes too low.

For the aforementioned reasons, enzyme-based methods are so far not common in the field of the production of glycopeptides.

The present invention relates to a method for the production of a glycosylated peptide comprising:
(i) providing a peptide conjugated with one or more hydrophilic polymer(s);
   and
(ii) glycosylating the polymer-conjugated peptide of step (i) by means of one or more glycosyltransferase(s).

As shown in the examples, it was found that peptides conjugated with a hydrophilic polymer could be very well glycosylated by means of glycosylating enzymes. Further, it was found that these peptides conjugated with a hydrophilic polymer could be purified easily. Therefore, it was possible to use the obtained polymer-conjugated peptides in several cyclical glycosylating-purifying steps. Herein, typically, each cycle comprised one glycosylating step and one purification step.

As used herein, the terms "glycosylated peptide" and "glycopeptide" may be understood interchangeably in the broadest sense as any peptide that comprises one or more carbohydrate moiety/moieties. Preferably, the glycosylated peptide is a peptide that is glycosylated in a site-specific and/or stereospecific manner, more preferably in a site-specific and stereospecific manner.

As used herein, the terms "carbohydrate moiety", "saccharide moiety" and "sugar moiety" may be understood interchangeably in the broadest sense as a molecular residue of any carbohydrate known in the art. A carbohydrate moiety may comprise of one or more monosaccharide unit(s).

A monosaccharide unit may be any monosaccharide unit known in the art, such as, e.g., a triose (e.g., an aldotriose (e.g. glyceraldehyde) or a ketotriose (e.g., dihydroxyacetone)), a tetrose (e.g., an aldotetrose (e.g., erythrose, threose) or a ketotetrose (e.g., erythrulose)), a pentose (e.g., an aldopentose (e.g., ribose, deoxyribose, arabinose, xylose, lyxose) or a ketopentose (e.g., ribulose, xylulose)), a hexose (e.g., an aldohexose (e.g., allose, altrose, glucose, mannose, gulose, idose, galactose, talose), other hexoses (e.g., glucuronic acid, galacturonic acid, N-acetylglucosamine/N-acetylchitosamine, glucosamine/chitosamine, N-acetylgalactosanine/N-acetylchondrosamine, fucose, desoxygalactose, rhamnose, deoxymannose, chinovose, deoxyglucose) or a ketohexose (e.g., fructose)) or a higher monosaccharide unit such as, e.g., heptulose, sedoheptulose, desoxymannooctulosonic acid/ketodesoxyoctonic acid, sialic acid/ N-acetylneuraminic acid.

Each monosaccharide unit may be a D- or an L-saccharide and may form a linear or cyclic (e.g., pyranose or furanose) structure. Optionally, one or more of the functional group(s) of a monosaccharide unit may be replaced by other residue(s) such as, e.g., amino group(s), carboxylic group(s), hydroxyl group(s) carbonyl group(s), hydrogen atom(s), halogen atom(s), alkyl group(s) (e.g., methyl group(s), ethyl group(s), propyl group(s)), aminoalkyl group(s) (e.g., aminomethyl group(s), aminoethyl group(s), aminopropyl group(s)), hydroxyalkyl group(s) (e.g., hydroxymethyl group(s), hydroxyethyl group(s), hydroxypropyl group(s)), one or more amino acid(s), lipid(s), lipid acid(s) and/or terpene(s). Preferably, the employed glycosyltransferase(s) can still accept the carbohydrate moiety of which one or more of the functional group(s) is/are replaced by one or more other residues(s) as a substrate.

Preferably, a monosaccharide unit is a pentose or a hexose, in particular a D-pentose or a D-hexose.

The carbohydrate moiety may be composed of one, two, three, four, five or more monosaccharide unit(s). The carbohydrate moiety may be a linear, branched or cyclic carbohydrate moiety. It may also be branched more than one times or may bear a symmetric or asymmetric dendrimer-like structure.

When there are more than one monosaccharide units, the monosaccharide units may be conjugated with another by any bond known in the art such as, e.g., an ether bond (R₁-OR₂), an amide bond R₁-CO-NH-R₂), a secondary amine (R₁-NH-R₂)), an imine R₁=N-R₂), an ester bond (R₁-CO-O-R₂), an alkyl linker (R₁-[CH₂]ₙ-R₂), a carbonyl group (R₁-CO-R₂), an alkenyl group (e.g., R₁=CH-R₂, R₁=CH-[CH₂]ₙ-R₂ or R₁-[CH₂]ₙ=CH-[CH₂]ₙ-R₂),), an alkynyl group (e.g., R₁=C-R₂, R₁=C-[CH₂]ₙ-R₂ or R₁-[CH₂]ₙ≡CH-[CH₂]ₙ-R₂), a thioether bond (R₁-S-R₂), a thioester bond (R₁-CO-S-R₂) or a thionoester bond (R₁-CS-O-R₂). As used herein, the residues R₁ and R₂, interchangeably, represent two carbohydrate moieties. It will be understood that the aforementioned bonds also comprise the charged and/or tautomeric forms thereof such as, e.g., R₁-N=C(OH)-R₂, R₁-NH₂⁺-R₂, or R₁-NH⁺=-R₂.

Preferably, the monosaccharide units may be conjugated with another by an ether bond, a secondary amine, an ester bond or an amide bond.

A glycopeptide of the present invention may bear one or more glycosylation site(s). As used herein a glycosylation site may be understood in the broadest sense as an amino acid moiety to which one or more saccharides may be conjugated with. In particular, a glycosylation site may refer to an amino acid moiety in a sequential context of amino acids, typically, a sequential context of a consecutive amino acid strand, to which one or more carbohydrate moiety/moieties is/are conjugated in nature. The glycosylation pattern of the glycopeptide of the present invention may be equal to the naturally occurring glycosylation pattern or may be different.

As used herein, the term "glycosylation pattern" may be understood in the broadest sense as the location and number of glycosylated sites of the peptide, the type(s) of conjugated monosaccharide(s), the number of conjugated monosaccharide(s) and/or the sequential orientation of the conjugated monosaccharide(s) in the carbohydrate moiety/moieties.

As used in the context of the present invention, the terms "conjugated with", "conjugated to" or "bound to" may be understood interchangeably in the broadest sense as the covalent linkage of two or more molecular moieties with another.

Typically a carbohydrate moiety is conjugated with a peptide via an ether bond (R₁-O-R₂), an amide bond R₁-CO-NH-R₂), a secondary amine (R₁-NH-R₂)), an imine R₁=N-R₂), an ester bond (R₁-CO-O-R₂), a alkyl linker (R₁-[CH₂]ₙ-R₂), a carbonyl group (R₁-CO-R₂), an alkenyl group (e.g., R₁=CH-R₂, R₁=CH-[CH₂]ₙ-R₂ or R₁-[CH₂]ₙ=CH-[CH₂]ₙ-R₂),), an alkynyl group (e.g., R₁≡C-R₂, R₁≡C-[CH₂]ₙ-R₂ or R₁-[CH₂]ₙ≡CH-[CH₂]ₙ-R₂), a thioether bond (R₁-S-R₂), a thioester bond (R₁-CO-S-R₂), or a thionoester bond (R₁-CS-O-R₂). As used herein, the residues R₁ and R₂, interchangeably, represent the carbohydrate moiety and the peptide, respectively. It will be understood that the aforementioned bonds also comprise the charged and/or tautomeric forms thereof such as, e.g., R₁-N=C(OH)-R₂, R₁-NH₂⁺-R₂, or R₁-NH⁺=-R₂.

Preferably, a carbohydrate moiety may be conjugated to an asparagyl or glutamyl residue via an amide bond or an ester bond, to a serinyl or threonyl residue via an ether bond, or to a cysteinyl residue via a thioether bond.

The term "peptide" as used in the context of the present invention may be understood in the broadest sense as a molecule comprising two or more amino acids that are conjugated with another via an amide bond. Preferably, the peptide is mainly composed of amino acids. In the context of peptides, an amide bond may also be designated as "peptide bond" or "peptidic bond". A peptide of more than two amino acids may also be designated as "polypeptide". A peptide may be a linear, cyclic or branched peptide. A peptide may comprise 2, 3, 4, 5, 6, 7, 8, 9, 10, more than 10, more than 15, more than 20, more than 25, more than 30, more than 40, more than 50, more than 75, more than 100, more than 200 or even more than 500 amino acids. A peptide that bears more than one secondary structure element and/or a tertiary structure element may also be designated as "protein domain" or "protein". Preferably, the peptide is a small peptide of less than 100 amino acids.

As used herein, the peptide preferably consists of a single amino acid strand. Alternatively, the peptide may also comprise two or even more strands covalently conjugated with another by any means such as e.g., by one or more disulfide bond(s).

Preferably, the peptide consists of natural L-amino acids. However, the peptide may also comprise one or more non-natural amino acid(s) such as, e.g., D-amino acid(s), beta amino acid(s), methylated amino acid(s) (e.g., N-methylated amino acid(s)). The peptide may even only consist of non-natural amino acids. The peptide may also be a *retro-inverso* peptide, thus a peptide mainly comprising D-amino acids and a reverse amino acid sequence compared to the corresponding naturally occurring peptide consisting of L-amino acids. As used in the context of the amino acid sequence, the term "naturally occurring" may be understood as a sequence that has at least 70 %, preferably at least 80 %, more preferably at least 90 %, even more preferably at least 95 % and most preferably 100 % sequence identity with the amino acid sequence found in nature.

The amino acid moieties may be positively charged, negatively charged or neutral. Also the whole peptide may be positively charged, negatively charged or neutral. The peptide may be hydrophilic or hydrophobic. The peptide may be well-water soluble or poorly water-soluble. As used herein, the term "poorly water-soluble" refers to a peptide which solubility is less than 5 mg/ml, less than 4 mg/ml, less than 3 mg/ml, less than 2 mg/ml, less than 1 mg/ml, less than 0.5 mg/ml or even less than 0.25 mg/ml peptide per water.

It will be understood by a person skilled in the art that the peptide may further bear any counter ion(s) known in the art, such as e.g., chloride ion(s), acetate ion(s), carbonate ion(s), hydrocarbonate ion(s), sodium ion(s), potassium ion(s), magnesium ion(s), and any ion(s) of the cleavage solution (e.g., TFA ion(s), bromide ion(s), perchlorate ion(s), ammonium ion(s)). Further, the peptide may be covalently or non-covalently associated to traces of one or more scavenger(s), such as, e.g., triisopropylsilane (TIS), dithiothreitol (DTT), anisole, thioanisole or 1,2-ethanedithiol.

Optionally, the peptide of the present invention may further bear one or more modification(s). The peptide may be amidated or capped at its C-terminus (e.g., by a non-amino acid moiety auch as, e.g., a bead and/or a polymer, in particular a hydrophilic polymer). As used herein, the terms "C-terminus", "C-terminal end", "carboxy terminus", "carboxyterminus" and "carboxyterminal end" may be understood interchangeably. Further, the N-terminus may be capped (e.g., by an acetyl moiety, a methyl moiety, a pyroglutamyl moiety, a bead and/or or a polymer, in particular a hydrophilic polymer). As used herein, the terms "N-terminus", "N-terminal end", "amino terminus" and "amino terminal end" may be understood interchangeably. The capping and/or amidation at one terminus or both termini may render the peptide more stable against exopeptidases. As used herein, preferably, the N-terminus of the peptide is capped by a hydrophilic polymer.

Optionally, one or more amino acid residue(s) of the peptide may be lipidated, phosphorylated, sulfated, cyclized, oxidated, reduced, decarboxylated, acetylated, acylated, amidated, deamidated, biotinylated or bound to one or more other small molecule(s) and/or terpene(s). Further, the peptide may or may not form one or more intramolecular disulfide bond(s) or one or more intermolecular disulfide bond(s).

Optionally, the glycopeptide of the present invention may be labeled radioactively (e.g., by ³H, ³²P, ³⁵S, ¹⁴C, ^{99m}Tc or lanthonoids (e.g., ⁶⁴Gd)) or may be labelled with a spin label, such as one or more heavy isotopes, e.g., ¹³C, detectable by Nuclear Magnetic Resonance (NMR).

Optionally, the glycopeptide of the present invention may be labeled by one or more small molecule dye(s) (e.g., Cy dye(s) (e.g., Cy3, Cy5, Cy5.5, Cy7), Alexa dye/s (e.g., Alexa Fluor 488, Alexa Fluor 546, Alexa Fluor 647, Alexa Fluor 680, Alexa Fluor 750), VisEn dye(s) (e.g. VivoTag680, VivoTag750), S dye(s) (e.g., S0387), DyLight fluorophore(s) (e.g., DyLight 750, DyLight 800), IRDye(s) (e.g., IRDye 680, IRDye 800), fluorescein dye(s) (e.g., fluorescein, carboxyfluorescein, fluorescein isothiocyanate (FITC)), rhodamine dye(s) (e.g., rhodamine, tetramethylrhodamine (TAMRA)) or HOECHST dye(s)) or one or more quantum dot(s).

As used throughout the present invention, the term "providing a peptide" may be understood in the broadest sense as obtaining a peptide from any source. The peptide may be provided by chemical synthesis, obtained from a biotechnological method and/or extracted from a natural source. Preferably, the peptide is provided by chemical synthesis. As used herein, the term "chemical synthesis" may refer to SPPS (Merrifield, 1963; Carpino and Han, 1972), liquid phase peptide synthesis or a combination of both. Here, the synthesis typically bases on the stepwise coupling of amino acids bearing protected side chains (orthogonal protecting groups). Typically, during synthesis, the peptide strand grows from the C-terminus to the N-terminus. However, there are alternative methods wherein the peptide strand grows from the N-terminus to the C-terminus. Nowadays, the most common methods base on at least two different types of protecting groups that are cleavable under at least two different conditions, such as, e.g., the fluorenyl-9-methoxycarbonyl/tert-butanyl- (Fmoc/tBu) protecting group scheme (Sheppard Tactics) or the tert-butoxycarbonyl/benzyl- (Boc/Bzl) protecting group scheme (Merrifield Tactics). Alternatively or additionally, the peptide may be also provided by conjugating two or more peptide strand(s) with another by any conjugation method known in the art such as, e.g., Native Chemical Ligation (NCL), Click Chemistry, Maleimide-Thiol Conjugation, enzymatic conjugation, biochemical protein ligation and/or soluble handling conjugation.

Alternatively, the peptide may be obtained from a biotechnological method. Today, numerous biotechnological methods are well-known in the art such as, e.g., overexpression and/or heterologous expression, in particular heterologous expression based on cloning of one or more gene(s) in bacteria, insect cells, mammalian cells or yeast cells. The peptide may further be extracted by any means known in the art. Alternatively, the peptide may be extracted from a natural source by any means known in the art.

Additionally, the peptide may be purified by any means known in the art, such as, e.g., one or more chromatographic method(s), one or more filtration method(s), one or more electrophoretic method(s), one or more precipitation-based method(s), one or more dialysis method(s) or a combination of two or more thereof. The natural source may be any biological material such as, e.g., bacterial material, plant material, animal material or fungal material, such as e.g. tissue, liquids or secretion(s). The peptide obtained from a natural source may also be digested or partly digested by one or more protease(s).

It will be understood by a person skilled in the art, that the aforementioned methods for providing a peptide may also be combined with another. In particular a peptide obtained from a biotechnological method or a natural source may further be purified and/or modified by chemical means known in the art.

As shown in the examples, the peptide is conjugated with one or more hydrophilic polymer(s).

The peptide conjugated with one or more hydrophilic polymer(s) may bear a lower tendency to interact with surfaces (e.g, plastic surfaces, glass surface and/or surfaces of column matrices) than the corresponding peptide not conjugated with one or more hydrophilic polymer(s). As used herein, interaction may be understood in the broadest sense as the stickiness and/or retention ability of the peptide and peptide conjugated with one or more hydrophilic polymer(s), respectively, when attached to a surface.

The term "hydrophilic polymer" as used herein may be understood in the broadest sense as any polymeric molecule that has a hydrophilic surface. A polymeric molecule comprises more than one monomer. The hydrophilic polymer may have a molecular mass of more than 500 Da, more than 1,000 Da, more than 1,500 Da, more than 2,000 Da, more than 5,000 Da or more than 10,000 Da. The hydrophilic polymer may be a soluble polymer, a gel-like polymeric matrix, a bead, a surface, a bead coating or the coating of a surface. Preferably, the hydrophilic polymer of the present invention is a soluble polymer.

Exemplarily, the hydrophilic polymer of the present invention may be polyethylene glycol (PEG) or derivatives thereof, polyethylene imine (PEI) or derivatives thereof, polyacrylic acid or derivatives thereof, in particular hydroxypropyl methacrylate (HPMA), polysaccharide(s) or derivatives thereof, preferably amino polysaccharide(s), aminoalkyl polysaccharide(s), hydroxyalkyl polysaccharide(s) and/or alkyl polysaccharide(s), lipopolysaccharide(s), hydrophilic polypeptide(s) and/or conjugate(s) or blockpolymer(s) comprising two or more of the aforementioned.

Alternatively, the hydrophilic polymer may be a bead having a hydrophilic surface or a bead having a coating comprising a hydrophilic polymer, in particular wherein the bead is a micro- or a nanobead. Alternatively, the hydrophilic polymer may be a solid material or a surface coating comprising a hydrophilic polymer. In this context, the hydrophilic polymer may optionally be the coating of a peptide microarray, the coating of a column matrix, in particular an affinity column matrix or the coating of an implant.

The hydrophilic polymer of the present invention may be conjugated with the peptide of the present invention via any functional group of the peptide. The hydrophilic polymer may be conjugated with the N-terminus of the peptide, an amino acid residue side chain, or the C-terminus of the peptide.

Depending on the polymer and the functional group of the peptide it is conjugated with, any conjugation strategy may be used, such as, e.g., formation of an ester bond, formation of an amide bond, formation of a thiolester bond, formation of an ether bond, formation of a thioether bond or formation of a disulfide bond.

Preferably, the hydrophilic polymer is conjugated with the N- or the C-terminus of the peptide, more preferably the hydrophilic polymer is conjugated with the N- or the C-terminus of the peptide via the formation of an amide bond, more preferably the hydrophilic polymer is conjugated with the N-terminus of the peptide via the formation of an amide bond.

Most preferably, the polymer is a PEG polymer conjugated with the N-terminus of the peptide via the formation of an amide bond.

In order to facilitate conjugation, a functional group of the peptide and/or a functional group of the polymer may be activated by any means known in the art such as, e.g., by one or more active groups(s).

As used throughout the invention, the terms "active group" may be understood in broadest sense as a molecule facilitating the formation of a bond, in particular an amide bond. Exemplarily, an active group may be an active ester (e.g., succinimidyl ester, N-hydroxysuccinimidyl (NHS) ester, carboxylic acid halogenide (acyl halide), pentafluorophenylester, thiophenyl ester, cyanomethyl ester, a nitrophenyl ester (e.g., 2-nitrophenyl ester, 4-nitrophenyl ester, 2,4-dinitrophenyl ester), a chlorophenyl ester (e.g., 2,4,5,-trichlorophenyl ester, pentachlorophenyl ester), pentafluorophenyl ester, N-hydroxypiperidinyl ester, 8-quinolyl ester, 1-hydroxybenzotriazolyl (HOBt) ester, 7-aza-1-hydroxybenzotriazolyl, N-norbornene-2,3-dicarboximidooxy ester, ethyl-1-hydroxy-1H-1,2,3-triazole-4-carboxylate), formation of an acid anhydride, and/or one or more coupling agent(s) (e.g., a carbodiimide (e.g., dicyclohexylcarbodiimide (DCC) and diisopropylcarbodiimide (DIC), a phosphonium reagent (e.g., benzotriazol-1-yloxytris(dimethylamino)phosphonium hexafluorophosphate (BOP)) or an uranium-type reagent (e.g., HBTU, TBTU, HATU, HAPyU, HAMDU, HAPipU, HAMTU).

Alternatively, the peptide may be conjugated to the hydrophilic polymer by any other means known in the art such as, e.g., by Native Chemical Ligation (NCL), Click Chemistry, Maleimide-Thiol Conjugation, and/or employing two or more different types of orthogonal protecting groups (e.g., convergent SPPS).

As used in the context of the present invention, the term "polymer-conjugated peptide" may be understood in the broadest sense as a peptide to which a hydrophilic polymer has been conjugated. Optionally, the polymer-conjugated peptide may form a monodisperse dispersion.

Preferably, the peptide is conjugated with one polymer in the stoichiometry 1 : 1. However, it will be understood that, optionally, more than one hydrophilic polymer may be conjugated with one peptide strand. Further, optionally, more than one peptide strands may also be conjugated with one hydrophilic polymer. Then, the hydrophilic polymer is "decorated" with peptides.

As shown in the examples, the polymer-conjugated peptide is further subjected to one or more glycosylating step(s) by means of one or more glycosyltransferase(s).

As used herein, the term "glycosylating" may be understood in the broadest sense as the conjugation of one or more carbohydrate moiety/moieties to the peptide. Preferably, in a first glycosylation step a glycosyltransferase specific for a particular carbohydrate moiety is used to conjugate said carbohydrate moiety with the peptide. After the glycosylation step, said glycosyltransferase may be removed. Optionally, one or more further glycosylation step(s) are conducted. As used herein, a glycosylation step may be understood as a reaction cycle.

In each one glycosylation step, a further glycosyltransferase specific for the conjugation of a further carbohydrate moiety is used to conjugate said further carbohydrate moiety with the peptide or with a carbohydrate moiety already conjugated with the peptide. More preferably, in a further glycosylation step, a further glycosyltransferase specific for the conjugation of a further carbohydrate moiety is used to conjugate said further carbohydrate moiety with the carbohydrate moiety already conjugated with the peptide and, thereby, elongates said carbohydrate moiety conjugated with the peptide. Then, the carbohydrate moiety conjugated with the peptide grows with each glycosylation step.

Most preferably, in each one glycosylation step, a glycosyltransferase conjugates a further monosaccharide unit to the growing carbohydrate moiety conjugated with the peptide.

As used in the context of the present invention, the terms "glycosyltransferase", "glycosylating enzyme" and "glycosylation enzyme" may be understood interchangeably in the broadest sense as any enzyme that can conjugate one or more carbohydrate moiety/moieties to the peptide and/or to the one or more carbohydrate moiety/moieties conjugated to the peptide of the present invention. Typically, a glycosyltransferase is an enzyme of enzyme classification (EC) class 2.4 that acts as a catalyst for the transfer of one or more monosaccharide(s), disaccharide(s), trisaccharide(s), oligosaccharide(s) or polysaccharide(s) from activated donor molecules to specific acceptor molecules (i.e., the peptide). Glycosyltransferases using nucleotide diphospho-carbohydrates, nucleotide monophospho-carbohydrates and carbohydrate phosphates include hexosyltransferases (EC 2.4.1), pentosyltransferases (EC 2.4.2) and transferases of other glycosyl groups (EC 2.4.99). The glycosyltransferase of the present invention may be any glycosyltransferase known in the art such as members of the families of glucosyltransferases, glucuronosyltransferases, galactosyltransferases, galacturonosyltransferases, fucosyltransferases, mannosyltransferases, N-acetylglucosaminyltransferases and N-acetylgalactosaminyltransferases (EC 2.4.1), xylosyltransferases, arabinosyltransferases and ribosyltransferases (EC 2.4.2), and sialyltransferases (EC 2.4.99).

Typically, a glycosyltransferase catalyses a site-specific glycosylation. The glycosyltransferase(s) of the present invention may be O-glycosyltransferase(s), N-glycosyltransferase(s) or S-glycosyltransferase(s), preferably O-glycosyltransferase(s) or N-glycosyltransferase(s), more preferably O-glycosyltransferase(s).

In a preferred embodiment, the method of the present invention further comprises the step of removing the glycosyltransferase(s) and/or reaction component(s) of the step of glycosylating the polymer-conjugated peptide by means of one or more glycosyltransferase(s) (step (ii)). More preferably, the method of the present invention further comprises the step of removing the glycosyltransferase(s) and reaction component(s).

As used herein, the term "removing" may be understood in the broadest sense as the decrease of the content of glycosyltransferase(s) and/or reaction component(s) by any means. Preferably, removing means that the content of glycosyltransferase(s) and/or reaction component(s) is decreased at least 10fold, more preferably at least 50fold, even more preferably at least 100fold, even more preferably at least 250fold, even more preferably at least 500fold and most preferably at least 1,000fold in comparison to the concentration of the reaction batch of the preceding the step of glycosylating the polymer-conjugated peptide by means of one or more glycosyltransferase(s) (glycosylation batch).

As used herein, the term "reaction component" may be understood in the broadest sense as any component that may be used for performing step (ii) or occurs during the step (ii) of glycosylating the polymer-conjugated peptide. Said component(s) may be, e.g., one or more carbohydrate protecting group(s), one or more protected or unprotected carbohydrate moiety/moieties not conjugated with the peptide, one or more cofactor(s) suitable for the employed glycosyltransferase(s), one or more side product(s) generated by the glycosyltransferase(s) and/or one or more precursor(s) usable by the employed glycosyltransferase(s).

As used herein, a cofactor suitable for the employed glycosyltransferase(s) may be, but may not be limited to a nucleotide triphosphate (e.g., adenosine triphosphate (ATP), guanosine triphosphate (GTP), uracil triphosphate (UTP)), lipids, vitamin(s) (e.g., biotin) and/or metal ions (e.g., iron ions, cobalt ions, magnesium ions).

As used herein, a side product generated by the glycosyltransferase(s) may be, but may not be limited to phosphate, pyrophosphate, uracil diphosphate (UDP), cytosine diphosphate (CDP), guanosine diphosphate (GDP), uracil monophosphate (UMP), cytosine monophosphate (CMP), uracil and/or cytosine.

As used herein, a precursor usable by the employed glycosyltransferase(s) may be, but may not be limited to one or more glycosyl moiety/moieties such as, e.g., one or more carbohydrate-phosphate(s), one or more carbohydrate-pyrophosphate(s), one or more carbohydrate-triphosphate(s), one or more nucleotide-diphosphate carbohydrate(s) (e.g., a uracil-diphosphate carbohydrate(s) (UDP-carbohydrate(s)), guanosin-diphosphate carbohydrate(s) (GDP-carbohydrate(s))), nucleotidephosphate carbohydrate(s) (e.g., cytosine-monophosphate carbohydrate(s) (CMP-carbohydrate(s))), one or more lipidlinked carbohydrate donor(s) (e.g., terpenoid-linked carbohydrate donor(s) (e.g., dolichol-or polyprenol-linked carbohydrate donor(s))).

Further it may be understood that one or more buffer component(s) such as, e.g., salts, buffer substances (e.g., phosphate ions, hydrogenphosphate ions, TAPS (3-{[tris(hydroxymethyl)methyl]amino}propanesulfonic acid), Bicine (N,N-bis(2-hydroxyethyl)glycine), Tris(tris(hydroxymethyl)methylamine), Tricine (N-tris(hydroxymethyl)methylglycine), TAPSO ([N-Tris(hydroxymethyl)methylamino]-2-hydroxypropanesulfonic acid), HEPES (2-hydroxyethyl-1-piperazineethanesulfonic acid), TES (2-{[tris(hydroxymethyl)methyl]amino}ethanesulfonic acid), MOPS (3-(N-morpholino)propanesulfonic acid), PIPES (piperazine-N,N'-bis(2-ethanesulfonic acid), Cacodylate (dimethylarsinic acid), SSC (sodium citrate) and/or MES (2-(N-morpholino)ethanesulfonic acid)) may be removed.

Further it may be understood that one or more detergent(s) may be removed. As used herein, the term "detergent" may be understood interchangeably with "surfactant". A detergent may be every detergent used in the art such as, e.g., Triton (X-100, CF-54), Nonidet P-40, CHAPS (3-[(3-Cholamidopropyl)dimethylammonio]-1-propanesulfonate), a polysorbate (glyceryl laurate, polyoxyethylene glycol sorbitan alkyl ester, e.g., polysorbate 20, polysorbate 21, polysorbate 40, polysorbate 60, polysorbate 61, polysorbate 65, polysorbate 80, polysorbate 81, polysorbate 85, polysorbate 120), a poloxamer (block copolymer of polyethylene glycol and polypropylene glycol), ADS (ammonium dodecyl sulfate), SLES (sodium lauryl ether sulfate), SMS (sodium myreth sulfate), DSS (dioctyl sodium sulfosuccinat)e, PFOS (perfluorooctanesulfonate), perfluorononanoate, PFOA (perfluorooctanoate), perfluorobutanesulfonatem, sodium stearate, Sodium lauroyl sarcosinate, octenidine dihydrochloride, CTAB (cetyl trimethylammonium bromide), CTAC (cetyl trimethylammonium chloride), CPC (cetylpyridinium chloride), POEA (polyethoxylated tallow amine), BAC (benzalkonium chloride), BZT (benzethonium chloride), dimethyldioctadecylammonium chloride, DODAB (dioctadecyldimethylammonium bromide), cocamidopropyl hydroxysultaine, cocamidopropyl betaine, lecithin, a polyoxyethylene glycol alkyl ether, an octaethylene glycol monododecyl ether, a pentaethylene glycol monododecyl ether, a polyoxypropylene glycol alkyl ether, a decyl glucoside, lauryl glucoside, an octyl glucoside, a polyoxyethylene glycol octylphenol ether, a polyoxyethylene glycol alkylphenol ether, a polyoxyethylene glycol alkylphenol ether, nonoxynol-9, a glycerol alkyl ester or a sorbitan alkyl ester. Preferably, the one or more detergent(s) is/are Triton, Nonidet P-40, CHAPS, a polysorbate or a poloxamer.

Further, the reaction component(s) may also be residuals of the step of providing a peptide conjugated with one or more hydrophilic polymer(s) (step (i)) still present when performing the glycosylating step (step (ii)). Said component(s) may be, e.g., one or more amino acid protecting group(s), one or more protecting group(s) of the hydrophilic polymer(s), one or more coupling agent(s), one or more scavenger(s), one or more cleaving agent(s), one or more protected or deprotected amino acid(s) not conjugated with the peptide, one or more protected or deprotected hydrophilic polymer(s) not conjugated with the peptide, one or more cofactor(s) suitable for the employed of glycosyltransferase(s), one or more side product(s) generated by the glycosyltransferase(s) and/or one or more precursor(s) usable by the employed glycosyltransferase(s).

After the glycosylation, the glycosyltransferase(s) and/or reaction component(s) may be removed by any method(s) known in the art such as, e.g., one or more chromatographic method(s), one or more filtration method(s), one or more electrophoretic method(s), one or more precipitation-based method(s) and/or one or more dialysis method(s).

Preferably, the step of removing the glycosyltransferase(s) and/or reaction component(s) does not require any toxic agent(s) such as, e.g., acetonitrile and TFA. Therefore, purification may preferably be performed with an aqueous solution such as a buffer, more preferably a buffer suitable for the glycosyltransferase(s) of the next glycosylating step as shown in the examples.

Preferably, removing the glycosyltransferase(s) and/or reaction component(s) is performed by a combination of not more than three different methods, more preferably a combination not more than two different methods or even not more than a single method.

Preferably, removing the glycosyltransferase(s) and/or reaction component(s) is performed by a chromatographic method. As used throughout the present invention, a chromatographic method may be any chromatographic method known in the art such as, e.g., gel permeation chromatography (GPC), high performance liquid chromatography (HPLC), reversed phase HPLC (RP-HPLC), fast protein liquid chromatography (FPLC), Flash Chromatography (flash), Rapid Refluid Liquid Chromatography (RRLC), Rapid Separation Liquid Chromatography (RSLC), Ultra Fast Liquid Chromatography (UFLC), reversed phase UFLC (RT-UFLC), Ultra Performance Liquid Chromatography (UPLC) or reversed phase UPLC (RT-UPLC). The column may be a prepacked column and/or a spin column usable by the employment of a centrifugal force.

Most preferably, the step of removing the glycosyltransferase(s) and/or reaction component(s) after one glycosylating cycle is performed by a single chromatographic step and/or a single precipitating step. That means that only one column and/or one precipitation step is used. That may lead to higher yields of the glycosylated peptide in comparison to a multistep procedure in which more than one chromatographic steps and/or more than one precipitating steps are used. This leads to a particular improvement of yields when there are more than one glycosylation steps, thus, more than one glycosylating cycles.

When using a chromatographic method, the chromatographic matrix may preferably be a bead matrix. The beads may be spherical or may bear any other shape known in the art to conduct chromatographic matrix. The beads may be of any material known in the art to be useful for preparing chromatographic material such as, e.g., silica, sugar-based bead material (e.g., agarose, sepharose), plastic bead material (e.g., polystyrene). The beads may bear a neutral, a positive or a negative zeta potential. The column may be a flow-through device or may be used batch-wise.

Preferably, removing the glycosyltransferase(s) and/or reaction component(s) is performed by a chromatographic method using an aqueous buffer as solvent. More preferably removing the glycosyltransferase(s) and/or reaction component(s) is performed by gel permeation chromatography (GPC).

As used herein, the terms "gel permeation chromatography", "molecular sieve chromatography", "gel filtration" and "size exclusion chromatography" may be understood interchangeably in the broadest sense as a chromatographic method for separating molecules via size. Typically the GPC matrix has a pore size in which several molecules that fit in (i.e., the polymer-conjugated peptide) are retarded and that high-molecular weight components (i.e., glycosyltransferase(s)) pass by. It will be understood that in the context of the present invention the pore-size is selected accordingly. Preferably the GPC matrix has a hydrophilic surface such as, e.g., cross-linked polysaccharide material (e.g, a cross-linked dextran gel (e.g., Sephadex material such as, e.g., Sephadex G-10 or Sephadex G-15)).

Preferably, a chromatographic method is a fast chromatographic method such, e.g., a chromatographic method working with a pressure that is higher than ambient pressure. This pressure may be obtained by using a pump and/or by centrifuging the column. A person skilled in the art knows the higher pressure may typically lead to a higher flow rate. Consequently, the tailing of the elution profile is minimized and the peptide is obtainable faster, in a higher purity, a higher yield and higher concentrated. Undesired interaction with the column matrix and surface is minimized.

Most preferably, removing the glycosyltransferase(s) and/or reaction component(s) is performed by centrifuging a microspin GPC column as used in the examples.

In the context of the present invention, an aqueous buffer may be any aqueous buffer suitable for the glycosyltransferase(s) used in the context of the present invention such as, e.g., phosphate buffered saline (PBS), a HEPES buffer, a Tris buffer, a TAPS buffer, a Bicine buffer, a Tricine buffer, a TAPSO buffer, a TES buffer, a MOPS buffer, a PIPES buffer, a Cacodylate buffer, an SSC buffer, an MES buffer, an acetate buffer or culture medium. Typically, the aqueous buffer will bear a pH in a range of between 4 and 9, preferably in a range of between 5 and 9, more preferably in a range of between 6 and 8.

Alternatively or additionally, a filtration method may be used. As used throughout the present invention, a filtration method may be any filtration method known in the art, such as, e.g, dead-end filtration or cross-flow filtration. Filtration may be conducted batch-wise of in a continuous flow method. As used herein, the terms "cross-flow filtration", "crossflow filtration", "tangential flow filtration" or "tangential filtration" may be understood interchangeably. The filter may be of any material known in the context of filtration in the art, such as, e.g., plastic (e.g., nylon, polysterene), metal, alloy, glass, ceramics, cellophane, cellulose, or composite material. The filter may be hydrophobic or hydrophilic. The surface of the filter may be neutral or positively charged or negatively charged. Preferably, the filtration method is a filtration method using an aqueous buffer as solvent.

Further, alternatively or additionally, an electrophoretic method may be used. As used throughout the invention, an electrophoretic method may be any electrophoretic method known in the art such as, e.g., gel electrophoresis or capillary (CE) electrophoresis. Preferably, the electrophoretic method is an electrophoretic method using an aqueous buffer as solvent.

Further, alternatively or additionally, a precipitation-based method may be used. As used throughout the present invention, a precipitation-based method may be any precipitation-based method known in the art such as, e.g., salting in, salting out or precipitation by adding one or more organic solvents (e.g, acetone, diethylether, ethanol, methanol, dichloromethane). Typically, precipitation is performed by adding a solvent in which only one or some of the component(s) is/are well-soluble and the other component(s) is/are not or poorly soluble and precipitate. The supernatant and/or the pellet may be used further. Exemplarily, precipitation may be diethyl ether precipitation.

Further, alternatively or additionally, a dialytical method (dialysis) may be used. Dialysis is based on diffusion and osmosis, respectively, and is well-known in the art. Dialysis is widely used in protein purification and is also used to provide an artificial replacement for lost kidney function in people with renal failure. The membrane may be of any material, such as, e.g., plastic (e.g., nylon, polysterene), metal, alloy, glass, ceramics, cellophane, cellulose, or composite material. The membrane may be hydrophobic or hydrophilic. The surface of the membrane may be neutral or positively charged or negatively charged. Preferably, dialysis is dialysis based on an aqueous buffer as solvent.

In a preferred embodiment, the step of removing the glycosyltransferase(s) and/or reaction component(s) is performed by chromatography and/or by precipitating the polymer-conjugated peptide, preferably by gel permeation chromatography (GPC) and/or precipitating the polymer-conjugated peptide, more preferably by a combination of GPC and precipitating the polymer-conjugated peptide, in particular by a combination of microspin GPC and precipitating the polymer-conjugated peptide.

As described above, most preferably, chromatography is a single chromatographic step, thus, the use of a single column only.

As used herein, the term "microspin GPC" may be understood in the broadest sense as any spin column packed with a GPC matrix that is usable in a centrifugal tube and has a maximal loading volume of less than 15 ml, less than 10 ml, less than 5 ml, less than 4 ml, less than 3 ml, less than 2 ml, less than 1 ml, less than 0.5 ml, or less than 0.25 ml. The microspin GPC column may be used in a centrifuge and subjected to a centrifugal force of at least 100 x g, at least 1,000 x g, at least 2,500 x g, at least 5,000 x g, at least 7,500 x g, at least 10,000 x g, at least 15,000 x g, at least 25,000 x g, or at least at least 50,000 x g, depending on the GPC matrix. In a microspin column, the glycosylated peptide is in contact with the column matrix for a comparably short time. The person skilled in the art will understand that this may increase yield and purity of the eluted glycosylated peptide. A tailing in the elution profile is minimized.

In a preferred embodiment, the peptide of the present invention is a small peptide of less than 100 amino acids, preferably less than 50 amino acids, more preferably less than 40 amino acids, even more preferably less than 35 amino acids, more preferably less than 30 amino acids, even more preferably less than 25 amino acids, in particular less than 20 amino acids.

Herein, the peptide preferably bears a molecular mass of less than 10 kDa, more preferably less than 5 kDa, even more preferably less than 4 kDa, and most preferably less than 3 kDa.

In a further preferred embodiment, the peptide is
(i) synthesized on a solid support, in particular by means of Fmoc- or Boc-based solid phase peptide synthesis (SPPS); and/or
(ii) conjugated with one or more hydrophilic polymer(s) on a solid support.

Preferably, the peptide is synthesized on a solid support by means of Fmoc- or Boc-based SPPS and is conjugated with one or more hydrophilic polymer(s) on a solid support. More preferably, the peptide is synthesized on a solid support by means of Fmoc- or Boc-based SPPS and is conjugated with one or more hydrophilic polymer(s) on said solid support without being cleaved off said solid support between the Fmoc-based SPPS and the conjugating of one or more hydrophilic polymer(s). Most preferably, the peptide is synthesized on a solid support by means of Fmoc-based SPPS, is conjugated with one or more hydrophilic polymer(s) on said solid support without being cleaved off said solid support between the Fmoc-based SPPS and the conjugating of one or more hydrophilic polymer(s).

As used herein, the terms "solid support" and "resin" may be understood interchangeably in the broadest sense as any solid matrix known for peptide synthesis in the art. Typically the solid support is a plastic bead. The solid support may be, but may not be limited to, chloromethyl resin (Merrifield resin), 4-benzyloxybenzyl alcohol resin (Wang resin), (2,4-dimethoxy)benzhydrylamine resin (Rink amide resin), 2,4-dialkoxybenzyl resin (super acid-sensitive resin, SASRIN®), 2-chlorotrityl resin, α-chlorotritylchloride resin (Barlos resin), benzhydrylamine resin (BHA resin), chloromethyl resin, hydroxymethylbenzoic acid resin (HMBA resin), 4-hydroxymethyl-3-methoxyphenoxybutyric acid resin (HMPB resin), hydroxycrotonoyl aminomethyl resin (HYCRAM resin), MBHA resin, oxime resin, 4-(hydroxymethyl)phenylacetamidomethyl resin (PAM resin) and/or a resin with special cleavable linkers (e.g., photolabile linkers or safety-catch linkers).

These methods typically base on the use of orthogonal protecting groups cleavable under special conditions. A protection group may be any protection group known in the art such as, e.g., an amino-protecting group of the urethane type (e.g., benzyloxycarbonyl (Z), 4-methoxybenzyloxycarbonyl (Z(OMe)), 2-nitrobenzyloxycarbonyl (Z(2-NO₂)), 4-nitrobenzyloxycarbonyl (Z(NO₂)), chlorobenzyloxycarbonyl (Z(Cl), Z(2-Cl), Z(3-Cl), Z(2,4-Cl)), 3,5-dimethoxybenzyloxycarbonyl (Z(3,5-OMe), α,α-dimethyl-3,5-dimethoxybenzyloxycarbonyl (Ddz), 6-nitroveratryloxycarbonyl (Nvoc), 4-(phenyldiazenyl)-benzyloxycarbonyl (Pz), a-methyl-2,4,5-trimethylbenzyloxycarbonyl (Bic), 2-(biphenyl-4-yl)-2-propoxycamonyl (Bpoc), (4-phenylazophenyl)-isopropoxycarbonyl (Azoc), isonicotinyloxycarbonyl (iNoc), tert-butoxycarbonyl (Boc), 2-cyano-tert-butoxycarbonyl (Cyoc), 2,2,2-trichloro-tert-butoxycarbonyl (Tcboc), adamantyl-1-oxycarbonyl (Adoc), 1-(1-adamantyl)-1-methoxycarbonyl (Adpoc), isobornyloxycarbonyl (Iboc), fluorenyl-9-methoxycarbonyl (Fmoc), (2-nirofluoren-9-yl)methoxycarbonyl (Fmoc(NO₂)), 2-(4-toluenesulfonyl)-ethoxycarbonyl (Tsoc), methylsulfonylethoxycarbonyl (Msc), 2-(4-nitrophenylsulfonyl)ethoxycarbonyl (Nsc), 2-(tert-butylsulfonyl)-2-propenyloxycarbonyl (Bspoc), 1,1 -dioxobenzo[b]-thien-2-ylmethoxycarbonyl (Bsmoc), 2-/methylsulfonyl)-3-phenyl-2-propenyloxycarbonyl (Mspoc), allyloxycarbonyl (Aloc), 2-(trimethylsilyl)-ethoxycarbonyl (Teoc), triisopropylsilylethoxycarbonyl (Tipseoc), piperidinyloxycarbonyl (Pipoc), cyclopententyloxycarbonyl (Poc), 3-nitro-1,5-dioxaspiro[5.5]undec-3-ylmethoxycarbonyl (PTnm), 2-ethynyl-2-propyl-oxycarbonyl (Epoc)), a carboxy-protecting group of the ester type (e.g., methyl (Me), ethyl (Et), benzyl (Bzl), 4-nitrobenzyl (Nbz), 4-methoxybenzyl (Mob), 2,4-di methoxybenzyl (2,4-Dmb), o-chlorotrityl (Trt(2-Cl), pyrimidyl-4-methyl(4-picolyl (Pic), 2-toluene-4-sulfonyl)-ethyl (Tse), phenacyl (Pac), 4-methoxyphenacyl (Pac(OMe), diphenylmethyl (Dpm), tert-butyl (tBu), cyclohexyl (Cy), 1-adamantyl (1-Ada), 2-adamantyl (2-Ada), dicyclopropylmethyl (Dcpm), 9-phenylfluoren-9-yl (Pf), 9-fluorenylmethyl (Fm), 2-trimethylsilylethyl (TMSE), 2-phenyl-trimethyl-silyl (PTMSE), allay (Al), 4-{N-[1-(4,4-dimethyl-2,6-dioxocyclohexanylidene)-3-methylbutyl]-amino}benzyl (Dmab)), a thiol-protecting group (e.g., benzyl (Bzl), 4-methylbenzyl (Bzl(4-Me)), 4-methoxybenzyl (Mob), 2,4,6-trimethoxybenzyl (Tmb), diphenylmethyl (Dpm), trityl (Trt), tert-butyl (tBu), acetamidomethyl (Acm), trimethylacetamidomethyl (Tacm), 9-fluorophenylmethyl (Fm), tert-butylsulfanyl (StBu), 3-nitro-2-pyridylsulfanyl (Npys), allyloxycrbanylaminomethyl (Alocam), 9H-xanthen-9-yl (Xan)), an imidazole protecting group (e.g., benzyl (Bzl), 2,4-dinitrophenyl (Dnp), benzyloxycarbonyl (Bom), tert-butoxycarbonyl (Boc), adamantly-1-oxycarbonyl (Adoc), triphenylmethyl (Trt), diphenylmethyl (Dpm), pyridyldiphenylmethyl (Pdpm), 4-toluenesulfonyl (Tosyl, Tos), 4-methoxybenzenesulfonyl (Mbs), tert-butoxymethyl (Bum), allyl (Al), allyloxymethyl (Alom)), or a hydroxyl-protecting group (e.g., benzyl (Bzl), 2,6-dichlorobenzyl (Dcb), diphenylmethyl (Dbm), cyclohexyl (Cy), 2-bromobenzyloxycarbonyl (Z(2-Br)), tert-butyl (tBu), 1-benzyloxycarbonyl-amino-2,2,2-trifluoroethyl (Zte), methylthiomethyl (Mtm), allyl (Al), allylcarbonyl (Aloc)).

Preferably, during synthesis of the peptide, only the protecting group at the α-amino group is cleaved off. Alternatively, to obtain a branched peptide, also another protecting group may be selectively cleaved off.

In a further preferred embodiment, the peptide is further cleaved from the solid support before glycosylation.

Therefore, after synthesis is completed, the protecting groups are preferably all cleaved from the peptide and the peptide is further cleaved from the solid support. Depending on the protecting group, cleaving may be conducted by any means known in the art suitable for the cleavage of the used protecting group(s) such as, e.g, addition of TFA, TFA and dichloromethane (CH₂Cl₂) (TFA/ CH₂Cl₂), acetic acid (AcOH), hydrobromic acid (HBr) and AcOH (HBr/AcOH), hydrochloric acid (HCl), AcOH and magnesium perchlorate (MgClO₄), a base (e.g., NH₃, K₂CO₃, triethylamine), piperidine (e.g., 5-25% piperidine in dimethylformamide (DMF)), DBU, 2-aminoethanol, morpholino, NaOC₂H₅, nucleophils (e.g., tris(2-aminoethyl)amine), a palladium catalyst (Pd), TBAF, electrolysis, gassing with hydrogen under a palladium catalyst (H₂/Pd), photolysis, or a combination of two or more thereof. It will be understood by a person skilled in the art that a mixture for cleaving a protecting group or for cleaving the peptide of the resin may further comprise one or more scavenger(s) such as, e.g., TIS, 1,2-ethanedithiol, anisole, DTT, thioanisole. Further, the solution may comprise SiCl₄, CH₂Cl and/or H₂O.

As used herein, the term "cleaved off the solid support" may be understood in the broadest sense as the release of a peptide previously conjugated to a solid support. Cleavage may be performed by any means known in the art. Typically, cleavage of a peptide synthesized by Fmoc/tBu strategy is conducted by a TFA-containing solution comprising between 80 and 100 % (v/v) TFA and between 0 and 20 % scavenger(s) such as, e.g., TIS, DTT, anisole, thioanisole, DTT and/or 1,2-ethanedithiol. Further, the solution may comprise SiCl₄, CH₂Cl and/or H₂O. Exemplarily, the solution may comprise 92.5 % (v/v) TFA, 2.5 % (v/v) TIS, 2.5 % (v/v) 1,2-ethanedithiol and 2.5 % (v/v) H₂O, or a solution comprising 95.0 % (v/v) TFA, 2.5 % (v/v) TIS and 2.5 % (v/v) H₂O.

Optionally, the synthesis of the peptide may also be combinatorial peptide synthesis. Herein, a library of more than 5, more than 10, more than 50, more than 100, more than 1,000 or even more than 10,000 peptides of different sequences may be obtained. By a combinatorial approach, on the one hand, novel glycosylation sites may be detected and, on the other hand, a peptide library for screening may be generated. The combinatorial approach may be parallel synthesis, synthesis in teabags, synthesis on pins (e.g., polyethylene pins), synthesis on strips (e.g., synthesis on cellulose or plastic strips), light-directed synthesis, addressable synthesis, split an combine methods, encoding methods or combinations of two or more thereof.

Preferably, the hydrophilic polymer(s) are conjugated to the peptide on the solid support. Here, the peptide is still bound to the solid support, preferably covalently bound to the solid support, when the hydrophilic polymer(s) is/are conjugated with said peptide. The one or more functional group(s) of said peptide are deprotected and the one or more polymer(s) are conjugated therewith. As used in the context of the present invention, the term "deprotected" means that the protecting group(s) is/are cleaved off from the respective functional group they were previously conjugated with. The hydrophilic polymer(s) is/are preferably conjugated covalently with the peptide.

Preferably, the peptide conjugated with one or more hydrophilic polymer(s) (polymer-conjugated peptide) is cleaved off the solid support before glycosylation. Further, the polymer-conjugated peptide may be purified and/or dissolved in an aqueous solution before glycosylation. As used herein, the aqueous solution may typically be a solution suitable for the employed glycosyltransferase(s).

However, alternatively, the peptide may be glycosylated on the resin. Then, the peptide may preferably be conjugated with the resin via a spacer molecule of more than 10 Å, more than 20 Å, more than 50 Å, more than 100 Å, or even more than 200 Å.

In a preferred embodiment, the step of conjugating the peptide with one or more hydrophilic polymer(s) is performed by site-specific conjugation, in particular by
(i) Native Chemical Ligation (NCL);
(ii) Staudinger Ligation, in particular Click Chemistry;
(iii) Maleimide-Thiol Conjugation;
(iv) Oxime formation;
(v) Thiazolidin Ligation;
(vi) Keto Acid-Hydroxy Amino (KAHA) Ligation; and/or
(vii) employing two or more different types of orthogonal protecting groups, in particular convergent solid phase peptide synthesis (SPPS).

Native Chemical Ligation (NCL) is a well-known conjugation method to couple an N-terminal cysteinyl residue with a thiolester. Preferably, the hydrophilic polymer(s) will comprise one or more thiolester group(s) and the peptide may bear an N-terminal cysteinyl residue. This N-terminal cysteinyl residue may be the N-terminus of a linear peptide or one or more of the N-termini of a branched peptide, preferably the N-terminus of a linear peptide. Typically, NCL is conducted in an aqueous environment and catalyzed by a thiol such as, e.g., thiophenol, thioethanol, thioanisole and/or benzyl mercaptane. The advantage of NCL is the site-specificity to the N-terminal cysteinyl residue. This method is also site-specific for a fully deprotected peptide.

A Staudinger Ligation in the context of the present invention is preferably Click Chemistry, in particular an Azide-Alkyne Huisgen Cycloaddition. Here an azide residue may react with an alkylene residue. The advantage is a site-specific conjugation. The peptide may bear the azide or the alkylene residue and the hydrophilic polymer(s) the corresponding other group. This method is also site-specific for a fully deprotected peptide.

Maleimide-Thiol Conjugation, also known as Maleimide Coupling, is a method based on the thiol-specificity of maleimides. Preferably, the hydrophilic polymer(s) will bear one or more maleimidyl moiety/moieties specifically binding to cysteinyl residues of the peptide. This method is also site-specific for a fully deprotected peptide.

Oxime formation is a well-known method based on the formation of a peptide oxime. Thiazolidin Ligation is based on the reaction of an N-terminal cysteine residue with an aldehyde or keto moiety.

Alternatively, the hydrophilic polymer(s) may be conjugated with the peptide by the employment of two or more different types of orthogonal protecting groups, in particular convergent SPPS as described above. Here, only specific protecting groups of the peptide are selectively cleaved off from particular functional groups of particular amino acid residues. Then, the hydrophilic polymer(s) may be conjugated by standard methods known for coupling reactions (see above).

Alternatively, the hydrophilic polymer(s) may be conjugated with the peptide via the formation of a disulfide bond. Preferably, one or more thiol residue(s) of the hydrophilic polymer(s) may form a disulfide bond with one or more cysteinyl residue(s) of the peptide. The formation of a disulfide bond may be achieved by oxidizing of a solution comprising a mixture of the hydrophilic polymer(s) and the peptide, e.g., by purging the solution with oxygen and/or air. Then, the hydrophilic polymer(s) are easily cleavable from the peptide by reducing the peptide-polymer conjugate.

In a preferred embodiment, the one or more hydrophilic polymer(s) is/are selected from the group consisting of
(i) polyethylene glycol (PEG) or derivatives thereof;
(ii) polyethylene imine (PEI) or derivatives thereof;
(ii) polyacrylic acid or derivatives thereof, preferably methacrylic acid or derivative thereof, more preferably hydroxypropyl methacrylate (HPMA) or hydroxyethyl methacrylate (HEMA) or derivatives thereof, in particular N-(2-hydroxypropyl) methacrylamide (HPMA) or derivatives thereof;
(iv) polysaccharide(s) or derivatives thereof, preferably aminopolysaccharide(s), aminoalkyl polysaccharide(s), hydroxyalkyl polysaccharide(s) and/or alkyl polysaccharide(s);
(v) lipopolysaccharide(s)
(vi) hydrophilic polypeptide(s); and/or
(vii) conjugate(s) or blockpolymer(s) comprising two or more of the above, preferably the hydrophilic polymer is PEG, more preferably a PEG polymer of between 5 to 50 PEG units, even more preferably of between 10 to 40 PEG units, even more preferably of between 15 to 35 PEG units, in particular of between 20 to 30 PEG units.

More preferably, the hydrophilic polymer(s) is/are synthetical polymers such as, e.g., polyethylene glycol (PEG) or derivatives thereof, polyethylene imine (PEI) or derivatives thereof, polyacrylic acid or derivatives thereof, or polysaccharide(s) or derivatives thereof.

In a highly preferred embodiment, the hydrophilic polymer is PEG, most preferably PEG of 27 PEG units (PEG₂₇). For the purpose of conjugation, PEG may comprise a protecting group at one end, preferably a Boc-protecting group (Boc-PEG₂₇-COOH) or an Fmoc-protecting group (Fmoc-PEG₂₇-COOH), in particular an Fmoc-protecting group.

Alternatively, the hydrophilic polymer may be polyethylene imine (PEI) or derivatives thereof, more preferably PEI of between 5 to 50 PEI units, even more preferably of between 10 to 40 PEI units, even more preferably of between 15 to 35 PEI units, in particular of between 20 to 30 PEI units.

Further, the hydrophilic polymer may alternatively be polyacrylic acid or derivatives thereof, preferably methacrylic acid or derivative thereof, more preferably hydroxypropyl methacrylate (HPMA) or hydroxyethyl methacrylate (HEMA) or derivatives thereof, in particular N-(2-hydroxypropyl) methacrylamide (HPMA) or derivatives thereof. Further, there were several HPMA derivatives such as, e.g., HPMA polymers comprising a glycyl-glycyl-linker (GG-linker) and HPMA polymers comprising a cleavable glycyl-phenylalanyl-leucyl-glycyl-linker (GFLG-linker) commercially available. In particular, the GFLG-linker is cleavable by lysosomal proteases.

Further, the hydrophilic polymer may alternatively be polysaccharide(s) or derivatives thereof, preferably aminopolysaccharide(s), aminoalkyl polysaccharide(s), hydroxyalkyl polysaccharide(s) and/or alkyl polysaccharide(s). As used herein, a polysaccharide may be any polysaccharide known in the art such as, e.g, starch, chitosan, glycan, dextran, cellulose or a derivative thereof. A derivative of said polysaccharide may be a polysaccharide, wherein one or more hydroxyl group(s) and/or the carbonyl group(s) of are replaced by other residue(s) such as, e.g., amino group(s), carboxylic group(s), carbonyl group(s), hydrogen atom(s), halogen atom(s), alkyl group(s) (e.g., methyl group(s), ethyl group(s), propyl group(s)), aminoalkyl group(s) (e.g., aminomethyl group(s), aminoethyl group(s), aminopropyl group(s)), hydroxyalkyl group(s) (e.g., hydroxymethyl group(s), hydroxyethyl group(s), hydroxypropyl group(s)), one or more amino acid(s), lipid(s), lipid acid(s) and/or terpene(s).

Exemplarily, the polysaccharide may be an alkylated (e.g., methylated, ethylated or propylated) polysaccharide (e.g., methylcellulose, ethylcellulose, methyl dextran, ethyl dextran), an polysaccharide comprising one or more amino groups (e.g., aminodextran, amino cellulose, aminoglycan, chitosan, aminostarch), an oxidated or reduced polysaccharide (carboxycellulose, carboxydextran), a cyclic polysaccharide (e.g., cyclodextran), carboxymethyl cellulose (CMC), carboxymethyl cellulose (CEC), carboxypropyl cellulose (CPC), carboxy aminomethyl cellulose, carboxy aminoethyl cellulose carboxy aminomethyl dextran, carboxy aminoethyl dextran, hydroxymethyl cellulose, hydroxyethyl cellulose, hydroxypropyl cellulose, hydroxymethyl dextran, hydroxyethyl dextran or hydroxypropyl dextran). However, it will be understood that the hydrophilic polysaccharide may also be any other polysaccharide known in the art. The polysaccharide may further be conjugated with any other small molecule, such as, e.g., a fluorophore, biotin, a maleimide moiety, a thiol ester moiety, etc.

Alternatively, the hydrophilic polymer may be a lipopolysaccharide or a derivative thereof.

Alternatively, the hydrophilic polymer may be a hydrophilic polypeptide. The hydrophilic polypeptide may be any polypeptide that is well-soluble in an aqueous buffer. It may be a short peptide mainly composed of hydrophilic amino acid moieties of less than 100, less than 50, less than 20, or even less than 10 amino acid residues. The hydrophilic polypeptide may be conjugated to the peptide as a single building block or may be added stepwise, e.g., as the conjugation of single amino acid residues, preferably hydrophilic amino acid residues. Alternatively, the hydrophilic polypeptide may be a protein of more than 100, more than 200 or even more than 300 amino acid residues.

A hydrophilic polymer may also be a bead with a hydrophilic surface such as, e.g., a quantum dot comprising a hydrophilic surface coating.

A person skilled in the art will notice that also conjugate(s) or blockpolymer(s) comprising two or more of the above may serve as the hydrophilic polymer in the sense of the present invention.

In a highly preferred embodiment, the hydrophilic polymer is conjugated with the N- or C-terminus of the peptide, in particular the N-terminus of the peptide.

The hydrophilic polymer may preferably be conjugated with the N-terminus of the peptide via a covalent bond.

The hydrophilic polymer may be permanently conjugated with the peptide or may be cleaved from the peptide after it has been glycosylated.

In a further preferred embodiment, the method of the present invention further comprises the step of cleaving the hydrophilic polymer(s) from the glycosylated peptide by cleaving at a cleavable site located between the glycosylation site(s) and the hydrophilic polymer(s).

In an even more preferred embodiment, said cleavable site is
(i) an enzymatically cleavable site, preferably a proteolytic cleavage site, in particular a proteolytic cleavage site located in a sequence extension of the peptide;
(ii) a part of a photolabile linker; or
(iii) a part of a chemolabile linker.

More preferably, the cleavable site is an enzymatically cleavable site. The term "enzymatically cleavable site" may be understood in the broadest sense as any molecular structure that is recognizable and cleavable by any enzyme known in the art. The enzymatically cleavage site may be, but may not be limited to, an enzymatically cleavage site of a protease, an enzymatically cleavage site of an esterase or a ligase. More preferably, the enzymatically cleavage site is an enzymatically cleavage site of a protease (proteolytic cleavage site). Most preferably, proteolytic cleavage site is a proteolytic cleavage site located in the peptide strand between the hydrophilic polymer and the glycosylation site.

The term "proteolytic cleavage site" may be understood in the broadest sense as any molecular structure that is recognizable and cleavable by any protease known in the art. As used in the context of the present invention, the terms "protease", "proteolytic enzyme", "peptidase" and "peptidolytic enzyme" may be understood interchangeably. Exemplarily, the proteolytic cleavable site is a cleavage site of the tobacco etch virus (TEV) protease, Factor Xa protease, SUMO protease, tobacco vein mottling virus (TVMV) protease, 3C protease, a serine protease (e.g., cathepsin G, chymotrypsin B, chymotrypsin C, DPP6, DPP7, elastase-1, elastase-2, EMR1, EMR2, EMR3, EMR4, enteropeptidase, epitheliasin, HtrA1 peptidase, HtrA2 peptidase, KLK1, KLK2, KLK3/PSA, KLK4, KLK5, KLK6, KLK7, KLK8, KLK9, KLK10, KLK11, KLK12, KLK13, KLK14, KLK15, matriptase-2, matriptase, mesotrypsin, neurotrypsin, PEE, PEE form B, PIM1, plasma kallikrein, prosemin, prostasin, PRSS16, TESP2, testisin, TMPRSS3, TMPRSS4, TMPRSS5, TMPRSS13, Trypsin 1, Trypsin 2, trypsin C), a metallo protease (e.g., ADAM1, ADAM2, ADAM3A, ADAM3B, ADAM6, ADAM7, ADAM8, ADAM9, ADAM10, ADAM11, ADAM12, ADAM15, ADAM17, ADAM18, ADAM20, ADAM22, ADAM23, ADAM28, ADAM29, ADAM30, ADAM32, ADAM33, MMP1, MMP2, MMP3, MMP7, MMP8, MMP9, MMP10, MMP11, MMP12, MMP13, MMP14, MMP15, MMP16, MMP17, MMP19, MMP20, MMP21, MMP24, MMP25, MMP26, MMP27, MMP28), an aspartic protease (e.g., cathepsin D, cathepsin E, Memapsin-1, Memapsin-2, NAPA, pepsin A, Pepsinogen C, presenilin 1, PS-2, Renin), a cyteine protease (e.g., calpain-1, calpain-2, calpain-3, calpain-5, calpain-7, calpain-9, calpain-10, calpain-11, calpain-12, calpain-13, calpain-14, calpamodulin, Caspase 1, Caspase 2, Caspase 3, Caspase 4, Caspase 5, Caspase 6, Caspase 7, Caspase 8, Caspase 9, Caspase 10, Caspase 14, cathepsin B, cathepsin F, cathepsin H, cathepsin K, cathepsin L, cathepsin O, cathepsin S, cathepsin V, cathepsin W, cathepsin X, legumain, or a threonine protease (e.g, gamma-glutamyltransferase 1, gamma-glutamyltransferase 2, gamma-glutamyltransferase 5, gamma-glutamyltransferase-like 3, gamma-glutamyltransferase-like 4, proteasome alpha 1, proteasome alpha 2, proteasome alpha 3, proteasome alpha 4, proteasome alpha 5, proteasome alpha 6, proteasome alpha 7, proteasome beta 1, proteasome beta 2, proteasome beta 3, proteasome beta 4, proteasome catalytic subunit 1, proteasome catalytic subunit 1i, proteasome catalytic subunit 2, proteasome catalytic subunit 2i, proteasome catalytic subunit 3, proteasome catalytic subunit 3i, Taspase 1). The proteolytic cleavage site may be the cleavage site of an exopeptidase or an endopeptidase, preferably an endopeptidase. Typically, a proteolytic cleavage site is a consecutive sequence of two or more amino acids.

Most preferably, the proteolytic cleavable site is the cleavage site of a protease having a high sequence specificity such as, e.g., a cleavage site of the tobacco etch virus (TEV) protease, Factor Xa protease, SUMO protease, tobacco vein mottling virus (TVMV) protease or 3C protease.

Preferably, the protease usable in the context of the present invention is sequence specific for a consecutive sequence of two or more amino acids, more preferably sequence specific for a consecutive sequence of three or more amino acids, even more preferably sequence specific for a consecutive sequence of four or more amino acids. As used herein, the term "sequence specific" may refer to the attribute that the enzymatic activity is at least 10fold, more preferably at least 50fold, even more preferably at least 100fold and even more preferably at least 200fold higher for a specific amino acid sequence than for other amino acid sequences. However, the person known in the art will immediately know that in this context the sequence specificity is not restricted to a consecutive sequence of particular residues of amino acids, but may also refer to a consecutive sequence of amino acid residues that bear specific physicochemical properties such as, e.g., hydrophilic amino acid residues, hydrophobic amino acid residues, small amino acid residues, bulky amino acid residues, neutral amino acid, negatively charged amino acid residues, positively charged amino acid residues, aliphatic amino acid residues or aromatic amino acid residues. The proteolytic cleavage site may be located within the peptide sequence or may be a peptide sequence added to the peptide. An added sequence may then comprise 1, 2, 3, 4, 5, 6, 7, 8, or more amino acid residues that represent the proteolytic cleavage site and, optionally, further amino acid residue(s) representing spacer amino acids for the purpose to prevent sterical hindrance of the employed proteolytic enzyme, in particular when the hydrophilic polymer(s) and/or the peptide is comparably bulky.

Alternatively, the cleavage site may be a part of a photolabile linker. As used herein, the terms "photolabile linker" and "photocleavable linker" may be understood interchangeably in the broadest sense as any molecular structure that is cleavable by a photochemical reaction, by irradiation of light of a certain wavelength at a certain intensity. Several photolabile linkers are known in the art. The light may be of a certain wavelength in the ultraviolet (UV) spectrum (wavelength: 1-400 nm), in the visible spectrum (wavelength range: 400-800 nm) or in the infrared (IR) spectrum (wavelength range: 800-5,000 nm). Most photolabile linkers are cleavable by irradiation of light of a certain wavelength of the UV-A spectrum (wavelength range: 300-400 nm). Exemplarily, the photolabile linker may be an O-nitrobenzyl photolabile linker, a molecule comprising a 1-(2-nitrophenyl)ethyl, a 3-hydroxy-2-naphthalenemethanol ether, a 3-hydroxy-2-naphthalenemethanol ester and/or a 4,4'-dimethoxytrityl ether. Preferably, the photolabile linker(s) is/are located between the peptide and the hydrophilic polymer(s).

Alternatively, the cleavage site may be a part of a chemolabile linker. As used herein, the terms "chemolabile linker" and "chemocleavable linker" may be understood interchangeably in the broadest sense as any molecular structure that is cleavable under certain chemical conditions. The chemolabile linker may be any chemolabile linker known in the art. Exemplarily, the chemolabile linker may be a Safety-Catch Linker. The chemolabile linker may be cleaved under certain chemical conditions such as, e.g., oxidative conditions, reductive conditions, acidic conditions and/or basic conditions. Further, the linker may be feat sensitive. When the chemolabile linker comprises a disulfide bond, it may be cleaved by reducing the peptide-polymer conjugate by any means such as, e.g., by addition of a reducing agent (e.g., DTT) or in the reductive environment of cytoplasm.

However, the hydrophilic polymer(s) may also be stable and, thus, not cleaved from the peptide. It is well-known in the art that a hydrophilic polymer may stabilize the peptide in a biological matrix, such as, e.g., the blood stream, extracted blood, blood plasma, blood serum, lymph, cytosol, cell-lysate etc. Therefore, the hydrophilic polymer(s) may be used to improve solubility and/or stabilize the peptide of the present invention against proteolytic degradation in a pharmaceutical or analytical composition *used in vivo* or in a cell-culture-based *in vitro* experiment. Further, the hydrophilic polymer(s) may be used to improve solubility in an in vitro experiment such as, e.g., a binding or an enzyme kinetic experiment. Moreover, the hydrophilic polymer(s) may be used to enable the conjugation of the peptide to a surface, such as the surface of a bead (e.g., a micro or a nanobead usable *in vitro or in vivo),* a microarray (e.g., a peptide or a protein microarray), a column matrix and/or an implant.

As known by those skilled in the art, peptides directly obtained from chemical synthesis are typically comparably small peptides of less than 50 amino acids. Therefore, there are several methods described in the art how peptide strands may be conjugated with other peptides to obtain longer polypeptide strands or even proteins.

Therefore, in another preferred embodiment, the method further comprises the step of conjugating the peptide obtainable by the method of the present invention to another peptide.

In an even more preferred embodiment, conjugating of the peptide to another peptide is performed by
(i) Native Chemical Ligation (NCL);
(ii) Click Chemistry;
(iii) Maleimide-Thiol Conjugation;
(iv) enzymatic conjugation, preferably a ligase- or a peptidase-based conjugation, in particular a subtiligase-, a trypsin- or a chymotrypsin-based conjugation;
(v) biochemical protein ligation, in particular expressed protein ligation (EPL) or protein trans-splicing (PTS);
(vi) soluble-handle conjugation, in particular Picolyl Ester Method; Thioester-Forming Ligation; Template-mediated Ligation; Capture-Mediated Ligation; and/or
(vii) employment of two or more different types of orthogonal protecting groups, in particular convergent solid phase peptide synthesis (SPPS).

As used herein, the term "another peptide" may be understood in the broadest sense as any other peptide strand. The other peptide may be a peptide bearing the same sequence or a peptide bearing another sequence. Preferably, the other peptide is a peptide bearing another sequence. The other peptide may be a glycopeptide or a non-glycosylated peptide. The other peptide may be a peptide of less than 20 amino acids, more than 20 amino acids, more than 50 amino acids, more than 100 amino acids, more than 150 amino acids, more than 200 amino acids or even more than 250 amino acids. Preferably, the peptides are conjugated with another by head-to-tail, thus, the C-terminus of the glycopeptide of the present invention may be conjugated to the N-terminus of the other peptide or the N-terminus of the glycopeptide of the present invention may be conjugated to the C-terminus of the other peptide. Alternatively, the glycopeptide of the present invention may be conjugated to a side chain of an amino acid residue of another peptide. It will be understood that additionally further peptides may be conjugated with another by any means known in the art.

Preferably, the other peptide may bear a sequence derived from the same naturally occurring peptide as the glycopeptide of the present invention. Therefore by conjugating the glycopeptide of the present invention to the other peptide, the naturally occurring full-length peptide or a longer fraction of the full-length peptide may be obtained. The glycosylation pattern of the obtained fusion peptide may be equal to the naturally occurring glycosylation pattern or may be different.

Alternatively, the glycopeptide of the present invention may be conjugated to another functional peptide such as, e.g., a fluorescent protein (e.g., (enhanced) green fluorescent protein (e)GFP, red fluorescent protein (RFP), yellow fluorescent protein (YFP), cyan fluorescent protein (CFP) or mCherry), a cell-penetrating peptide (e.g., a polyarginine (e.g., R₇, R₈ or R₉), a penetratin peptide, a Chariot peptide, a lactoferrin-derived peptide, a HIV Tat peptide, a SynB1 peptide, a Buforin peptide, a Magainin peptide, a HIV-gp41 peptide or a Kaposi FGF signal sequence peptide), or a reporter group (e.g., a histidine (HIS) tag., streptavidin or a peroxidase). Depending on the fusion partner, the obtainable fusion peptide may then bear novel functions such as, e.g., fluorescence, cell-permeability, or binding to novel binding partners.

Exemplarily, the glycopeptide of the present invention may be conjugated to another peptide via Native Chemical Ligation (NCL). Here, the glycopeptide of the present invention may bear an N-terminal cysteinyl residue and may be conjugated to a thiolester located at the C-terminus of the other peptide. Alternatively, the other peptide may be conjugated to a thiolester located at the C-terminus of the glycopeptide of the present invention. Alternatively, the peptides may also be conjugated with another via a thiolester-containing side chain amino acid residue.

Alternatively, the glycopeptide of the present invention may be conjugated to another peptide via Click Chemistry. The glycopeptide of the present invention may bear the azide or the alkylene residue and the other peptide may bear the corresponding other group. Preferably, the azide residue is located at the C-terminus of the glycopeptide of the present invention and the alkylene residue is located at the N-terminus of the other peptide or the azide residue is located at the C-terminus of the other peptide and the alkylene residue is located at the N-terminus of the glycopeptide of the present invention.

Alternatively, the glycopeptide of the present invention may be conjugated to another peptide via Maleimide-Thiol Conjugation. The glycopeptide of the present invention may bear a maleimidyl moiety specifically binding to cysteinyl residue of the other peptide or the other peptide may bear a maleimidyl moiety specifically binding to cysteinyl residue of the glycopeptide of the present invention. Preferably, the maleimidyl moiety is located at the C-terminus of the glycopeptide of the present invention and the cysteinyl residue is located at the N-terminus of the other peptide or the maleimidyl moiety is located at the C-terminus of the other peptide and the cysteinyl residue is located at the N-terminus of the glycopeptide of the present invention.

Alternatively, the glycopeptide of the present invention may be conjugated to another peptide via enzymatic conjugation. As used herein, the term "enzymatic conjugation" may be understood in the broadest sense as any means of a ligation that is catalyzed by an enzyme. Preferably, the enzymatic conjugation is a ligase- or a peptidase-based conjugation. Herein, it is utilized that an enzymatic reaction is typically reversible. In particular, the enzymatic conjugation is a subtiligase-, a trypsin- or a chymotrypsin-based conjugation. Preferably, ligation is ligation of the C-terminus of the glycopeptide of the present invention to the N-terminus of the other peptide or the N-terminus of the glycopeptide of the present invention to the C-terminus of the other peptide.

Alternatively, the glycopeptide of the present invention may be conjugated with another peptide by biochemical protein ligation. In particular, biochemical protein ligation may be expressed protein ligation (EPL) (also: intein-mediated protein ligation (IPL)) or protein-trans splicing (PTS). Herein, naturally occurring inteins may be exploited to prepare a recombinant polypeptide C-terminal thiolester. This may then be conjugated via NCL with an N-terminal cysteinyl moiety as described above.

Alternatively, the glycopeptide of the present invention may be conjugated with another peptide by soluble-handle conjugation. In particular, soluble-handle conjugation may be Picolyl Ester Method; Thioester-Forming Ligation; Template-mediated Ligation; Capture-Mediated Ligation. Picolyl Ester Method is a method well-known in the art that bases on the orthogonal cleavability of a picolyl ester moiety. A picolyl ester moiety is comparably resistant to acids and may, therefore, be used as a semipermeable blocking group for the C-terminal carboxy group. Capture-Mediated Ligation may be, e.g., thiol capture ligation, reverse thioester ligation or imine capture ligation. These methods are well-known to those skilled in the art. Further, ligation may also be based on the formation of thiolester between the two peptides (R₁ and R₂) by any means such as, e.g., by the reaction of C-terminal thiocarboxy group (R₁-CO-SH) with an N-terminal halogen acetyl group (e.g., a bromoacetyl group (Br-CH₂-CO-R₂)). Further, ligation may also be based on the formation of a peptide hydrazone or of a peptide oxime by any means known in the art.

Alternatively, the glycopeptide of the present invention may be conjugated to another peptide by the employment of two or more different types of orthogonal protecting groups. Here only specific protecting groups of the glycopeptide of the present invention and the other peptide are selectively cleaved off. Then, the glycopeptide of the present invention may be conjugated by standard methods known for coupling reactions (see above). Alternatively, the glycopeptide of the present invention may be conjugated to another peptide by the formation of a disulfide bond. Preferably, a cysteinyl residue of the glycopeptide of the present invention may form a disulfide bond with cysteinyl residue of the other peptide. The formation of a disulfide bond may be achieved by oxidizing a solution comprising a mixture of the glycopeptides and the other peptide.

Further, it will further be understood that, alternatively or additionally, two or more peptides of the present invention may be combined with another and/or with one or more other peptide(s) on one polymeric backbone, in particular wherein said polymeric backbone is/are the hydrophilic polymer(s) of the present invention. In this case the peptides are all conjugated with the polymer(s), but do not necessarily be conjugated directly with another.

In a preferred embodiment, the method of the present invention further comprises the step of purifying the peptide, preferably by one or more chromatographic method(s) and/or one or more precipitation step(s), in particular gel permeation chromatography (GPC), fast protein liquid chromatography (FPLC), reversed phase high performance liquid chromatography (RP-HPLC) and/or diethyl ether precipitation.

The peptide may be purified by any means known in the art, e.g., by one or more chromatographic method(s), one or more filtration method(s), one or more electrophoretic method(s), one or more precipitation-based method(s) and/or one or more dialysis method(s). Preferably, purifying the peptide is performed by GPC, FPLC, RP-HPLC and/or diethyl ether precipitation. More preferably, purifying the peptide is performed by GPC, FPLC and/or RP-HPLC and diethyl ether precipitation.

As used herein, diethyl ether precipitation is preferably performed with cooled diethyl ether, such as, e.g., diethyl ether at 4°C, -20°C or -80°C. The cooled diethyl ether may be added to the glycopeptide-containing reaction broth. The peptide is then precipitated. Optionally, the reaction tube containing diethyl ether and peptide is incubated at a low temperature such as, e.g., at 4°C, -20°C or -80°C. A peptide-containing pellet may be obtained by centrifugating the reaction tube. The diethyl ether solution may optionally further contain other organic solvents such as, e.g., ethanol. Optionally, ethanol is first added to the reaction containing the peptide, mixed, and diethyl ether is added before incubation at a low temperature such as, e.g., at 4°C, -20°C or -80°C. The diethyl ether solution may be discarded and/or evaporated. The obtained peptide-containing pellet may be dried or dissolved in a suitable solvent (e.g., an aqueous buffer or an organic, polar solvent (e.g. dimethyl sulphoxide (DMSO), dimethyl formamide (DMF), acetonitrile, ethanol and/or methanol)). The peptide may first be purified by precipitation and subsequently purified by a chromatographic method or *vice versa.*

In the most preferred embodiment, the method of the present invention comprises
(i) synthesizing a peptide on a solid support;
(ii) conjugating a hydrophilic polymer to the peptide of step (i) on the solid support;
(iii) cleaving the polymer-conjugated peptide of step (ii) off the solid support;
(iv) purifying the polymer-conjugated peptide of step (iii);
(v) glycosylating the peptide by means of one or more glycosyltransferase(s);
(vi) removing the one or more glycosyltransferase(s) and/or one or more reaction component(s) of step (v), preferably by means of chromatography, more preferably by means of GPC, in particular by means of microspin GPC; and
(vii) optionally reconstituting the peptide for the next glycosylation step and repeating the steps (v) and (vi).

Preferably, after each gycosylation step the one or more glycosyltransferase(s) and/or one or more reaction component(s) and the reaction component(s) are removed by any means known in the art and described above. Optionally, the glycopeptide obtained from the precedent glycosylation step is precipitated and/or dried. The peptide may be stored and/or purified further by any means known in the art or may be subjected to a further glycosylating step. When the glycopeptides is subjected to a further glycosylating step, the peptide may then be dissolved in a solution suitable for the glycosyltransferase(s) of the next glycosylating step and the gycosylation step is conducted as described above. Then, a cycle is completed. This cycle may be repeated as often as desired by the experimenter. In each cycle, a further carbohydrate moiety is added to the growing carbohydrate moiety/moieties conjugated with the peptide. Alternatively, one or more other amino acid side chain(s) may be conjugated with carbohydrate moiety/moieties.

In another most preferred embodiment, the method of the present invention further comprises the step of proteolytic cleavage in the peptide strand between the hydrophilic polymer and the glycosylation site.

In a further preferred embodiment, the method of the present invention further comprises:
(i) precipitating the peptide, in particular by means of diethyl ether precipitation;
(ii) drying the peptide, and/or
(iii) preserving the peptide, preferably by freeze-drying, freezing, addition of one or more preservative agent(s) and/or dissolution of the peptide in an organic solvent.

The peptide may be precipitated after each glycosylation step and/or may be precipitated after all glycosylation steps have been completed. The precipitated peptide may further be centrifuged and/or filtrated to obtain a pellet comprising the peptide. Said pellet comprising the peptide or a filtrate of the peptide may be dried by any means known in the art (e.g., at normal pressure at underpressure (e.g., in an exicator, in a SpeedVac) or may be dissolved in any solution (e.g., in an aqueous buffer, in DMSO, in DMF, in ethanol, in methanol, in acetonitrile or in a mixture of two or more thereof).

As used herein, the term "preserving the peptide" may be understood in the broadest sense as any means for preparing a storable peptide and elongating shelf-life of said peptide. Exemplarily, preserving the peptide may be drying or freeze-drying. As used herein, the terms "freeze-drying", "lyophilization" and "cryodesiccation" may be understood interchangeable in the broadest sense as the removal of evaporable residuals from the peptide in the frozen state. Typically, the peptide is first dissolved in an aqueous solution, preferably in water, a water/acetonitrile mixture or a buffer with extensive volatile components. The sample is then frozen (e.g., preferably at -80° or liquid nitrogen) and, finally, the sample is dehydrated. The freeze-dried peptide may be stored at any suitable temperature such as, e.g., at room temperature, at -20°, at -80°C or in liquid nitrogen. Before use, the peptide may be dissolved in any suitable solvent (e.g., an aqueous buffer or an organic, polar solvent (e.g. DMSO, DMF, acetonitrile, ethanol, methanol or a mixture of two or more thereof)). Alternatively, the peptide may be frozen such as, e.g., at -20°, -80°C or in liquid nitrogen. The peptide may be frozen in the dry state or dissolved in a suitable solvent. Before use, the peptide may be thawed. The peptide may further be preserved by adding one or more preservative agent(s) such as, e.g., sodium azide (NaN₃) and/or benzoic acid. Further, the peptide may be dissolved in an organic solvent such as, e.g., DMSO, DMF, acetonitrile, ethanol, methanol or a mixture of two or more thereof. Further, in order to prevent oxidation of one or more of the peptide side chain(s) and/or the carbohydrate moiety/moieties, optionally, one or more reducing agent(s) may be added to the stored peptide such as, e.g. traces of a thiol.

The present invention further relates to a glycosylated peptide obtained from the method of the present invention or salts thereof, in particular pharmaceutically acceptable salts thereof.

As the process of glycosylating the polymer-conjugated peptide by means of one or more glycosyltransferase(s) is highly stereospecific, the glycosylated peptide obtained from the method of the present invention is typically an extensively pure stereoisomer. Typically, more than 50%, more than 70%, more than 80%, more than 90%, more than 95%, more than 97%, more than 98%, more than 99% or even 100% of the obtained peptide represent one stereoisomer. Typically, said glycosylated peptide will comprise L-amino acids and carbohydrate(s) composed of D-monosaccharide units. In contrast to peptides obtained by other methods, said glycosylated peptide will be extensively free of TFA, protecting groups and scavengers as TFA is merely used in a single step, namely, the optional step of cleavage of the peptide from the solid phase. Therefore, said glycosylated peptide is particularly well usable in for any means a glycosylated peptide may be used for, in particular for one or more pharmaceutical purpose(s).

Exemplarily, said glycosylated peptide or a pharmaceutically acceptable salt thereof may be used for a pharmaceutical purpose such as, e.g., as antibiotic, hormone, cytokine, vaccine, artificial extracellular matrix, artificial glycocalyx and/or coating for an implant.

Preferably, said glycosylated peptide pharmaceutically acceptable salt thereof may be used as a vaccine, in particular a mixed vaccine, thus, a vaccine in that several peptide moieties of a glycopeptide of the present invention and at least a part of the one or more carbohydrate moiety/moieties conjugated with said glycopeptide in common serve as an antigen. Said antigen may be recognizable by an immune cell receptor or an antibody or fragment thereof. Said glycosylated peptide may also serve as a haptene.

Said glycosylated peptide or a pharmaceutically acceptable salt thereof may be used in a method for the treatment or prevention of a disease such as, e.g., cancer, thrombosis, myocardial infarction, viral or bacterial infection and/or stroke. In particular, the glycosylated peptide of the present invention may be used in a method for the prevention of a viral or bacterial infection or for the treatment of cancer by immune therapy.

Further, said glycosylated peptide or a salt thereof may be used for scientific research, in particular research dealing with cell-cell interaction, cell-matrix interaction, neoplastic cells, cancer, thrombosis, myocardial infarction, viral or bacterial infection and/or stroke. The peptide obtainable by the method of the present invention may also be used in a screening library or on a microarray for detecting novel interaction partners of glycopeptides(s) or characterizing interaction pattern(s) of glycopeptides(s) with cells or other peptides. The peptide may further be immobilized on an affinity chromatographic matrix and used for affinity chromatography.

Further, said glycosylated peptide or a salt thereof may be a short mucin-type O-glycopeptide. Preferable, said mucin-type O-glycopeptide is carrying the Tn-, T- or STn-antigen or combinations thereof, in particular Tn-, T- or STn-antigen O-glycans or combinations thereof usable as a cancer vaccine.

The invention is further explained by the following examples and figures, which are intended to illustrate, but not to limit the scope of the present invention.

### Brief Description of the Figures

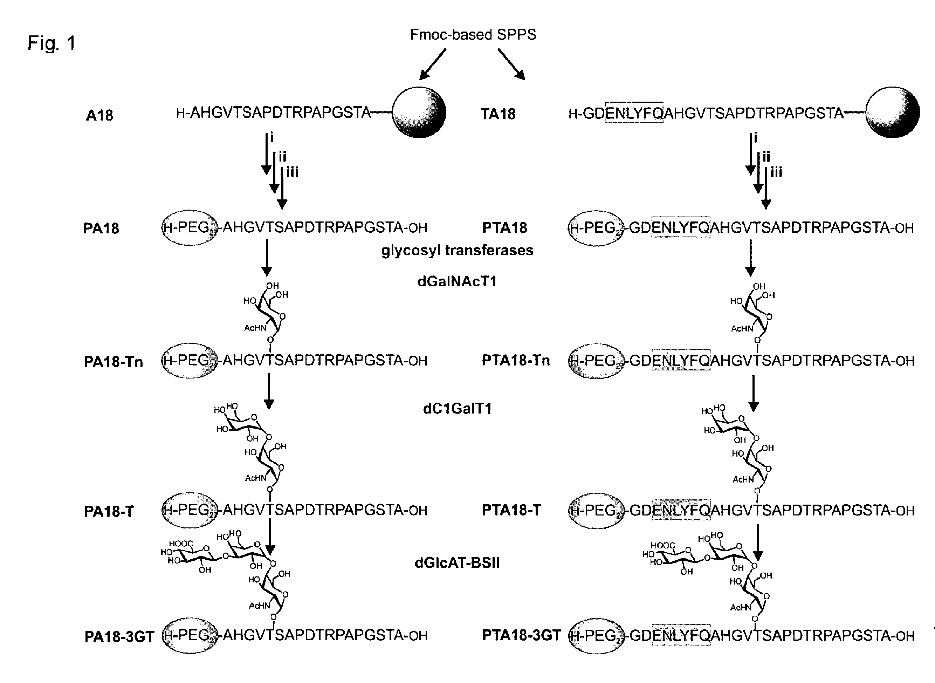

**Figure 1** illustrates the chemoenzymatic synthesis of monodisperse PEG₂₇-peptide conjugates of the MUC1 tandem repeat. Peptide A18 is accessed by SPPS and represents one MUC1 tandem repeat sequence that is subsequently modified with Fmoc-PEG₂₇-COOH in order to obtain PA18 (i, coupling of Fmoc-PEG₂₇-COOH; *ii*, removal of Fmoc group; iii, cleavage from solid support). Peptide TA18 is generated by extending A18 with 8 amino acids comprising a *TEV protease* cleavage site for traceless removal of the PEG₂₇-tag. PTA18 contains a similar N-terminal PEG₂₇-modification as described above for PA18. The glycoforms indicated by the suffix Tn, T and 3GT are obtained by enzymatic glycosylation with *dGalNAcT1, dC1GalT1* and *dGlcAT-BSII,* respectively.

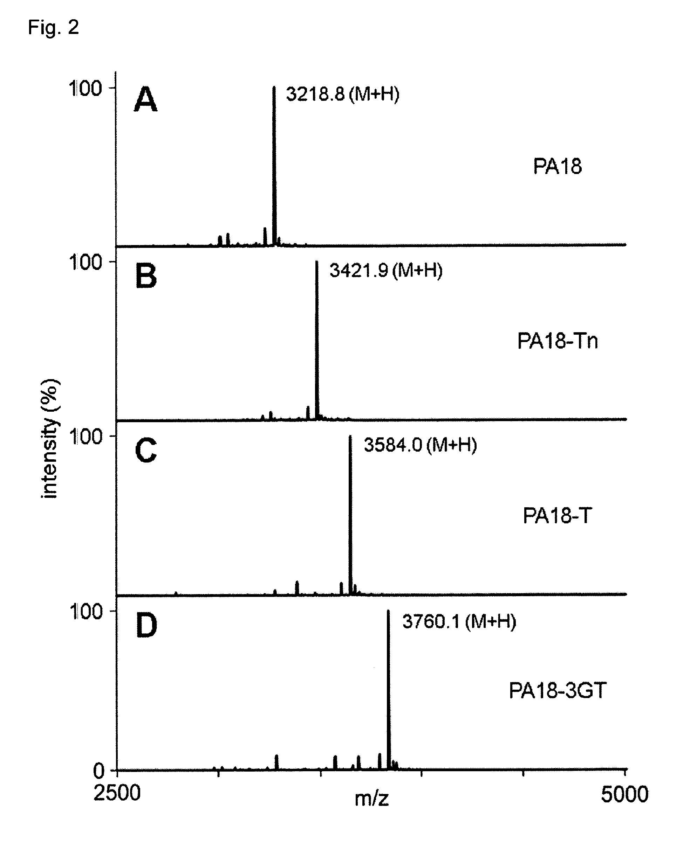

**Figure 2** illustrates the MALDI-MS of chemoenzymatically synthesized PA18 glycoforms. Monoisotopic masses (M+H) of synthetic (A) and enzymatically glycosylated PEG₂₇ peptide conjugates *(B-D)* are displayed. (*B*)*,* PA18 was quantitatively glycosylated by *dGalNAcT1* with a single GaINAc (PA18-Tn, Δm/z +203.1). (C), PA18-Tn was fully converted to PA18-T by enzymatic addition of galactose (Δm/z +162.1) by *dC1GalT1. (D),* Elongation of PA18-T to PA18-3GT was accomplished by quantitative glycosylation with *dGlcAT-BSII* adding terminal glucuronic acid (Δm/z +176.1). Traces of shortened PEG26-conjugates (Δm/z -44) are indicated by *asterisks.*

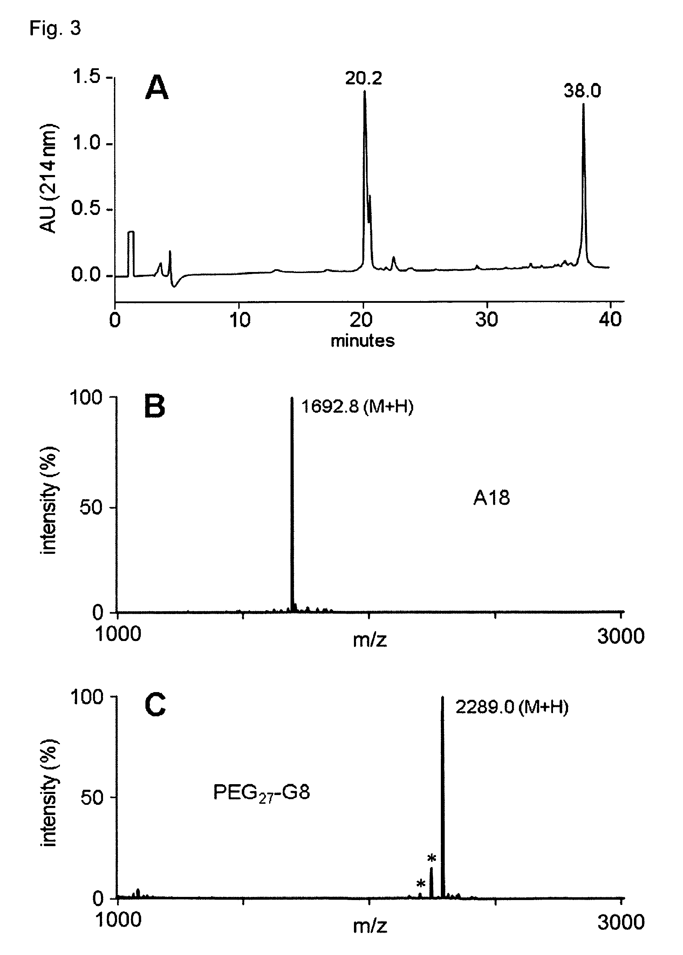

**Figure 3** illustrates the quantitative release of the MUC1TR peptide A18 by *TEV protease* digest of PTA18. (A) RP-HPLC (C18) profile of 50 µg PTA18 digested with Tobacco Etch Virus protease. (B) MALDI-TOF MS spectrum of A18 eluting at 20.2 min (22 % Eluent B). (C) MS spectrum of the N-terminal PEG₂₇-G8 eluting at 38.0 min (100 % Eluent B). Monoisotopic masses (M+H) of positive ions are indicated. Minute amounts of PEG26-G8 (Δm/z -44) and PEG25-G8 (Δm/z -88) are indicated by *asterisks.*

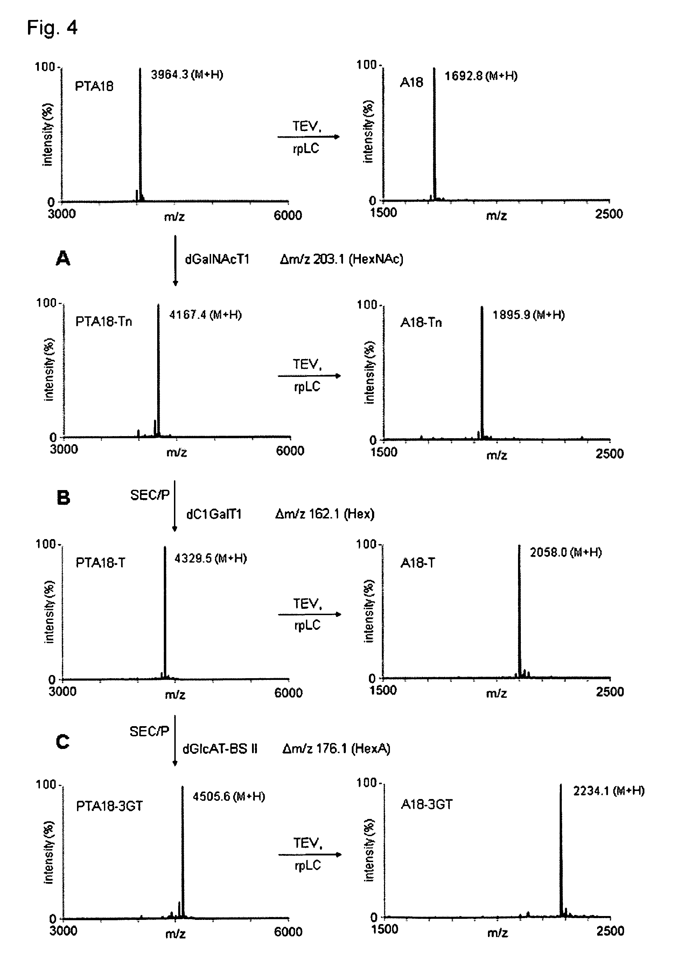

**Figure 4** illustrates the biosynthesis of A18 O-glycopeptides via enzymatic release from PTA18 precursors. *Left panel,* MALDI-TOF MS diagrams of stepwise enzymatic sugar additions to PTA18. (*A*) Quantitative glycosylation of PTA18 (m/z 3964.3) by *dGaINAcT1* generates PTA18-Tn (m/z 4167.4). (*B*)*, dClGalT1* extends PTA18-Tn to PTA18-T (m/z 4329.5). (C), The core 1 O-glycan of PTA18-T is extended to glucuronyl-T by *dGlcAT-BSII* forming PTA18-3GT (m/z 4505.6). *Right panel,* MALDI-TOF MS analyses of A18 glycopeptides released from the corresponding PTA18 glycoforms by *TEV protease* digest. *dGalNAcT1, Drosophila* polypeptidyl GalNAc-transferase 1 (pgant35A), *dClGalT1, Drosophila* core 1 β1-3-galactosyltransferase 1, *dGlcAT-BSII, Drosophila* β1-3-glucuronyltransferase BSII, *RP-LC,* reversed phase liquid chromatography, *SEC*/*P,* size exclusion chromatography/precipitation, *TEV,* tobacco etch virus protease. Monoisotopic masses (M+H) of positive ions are indicated.

### Examples

### Example 1:

### Chemical Synthesis of Monodisperse PEG₂₇-Peptide Conjugates Derived from the MUC1 Tandem Repeat

Fmoc-protected amino acids, 2-(1H-benzotriazol-1-yl)-1,1,3,3-tetramethyluronium hexafluorophosphate (HBTU) and preloaded Wang resins were purchased from Novabiochem. TFA was from Roth. Fmoc-PEG₂₇-COOH was from Polypure (Norway). All other chemicals were obtained from Sigma-Aldrich at the highest purity available.

### Peptide Synthesis

Solid phase peptide synthesis (SPPS) of mucin peptides (A18, H-AHGVTSAPDTRPAPGSTA-OH (SEQ ID NO:1)) and (PTA18, H-GDENLYFQAHGVTSAPDTRPAPGSTA-OH (SEQ ID NO:2))) was performed manually using standard Fmoc-deprotection procedures and HBTU-activated couplings (Merrifield, 1963; Carpino and Han, 1972). After cleaving the peptide from the resin using a mixture of 25 µl H₂O, 25 µl TIS and 950 µl TFA, it was precipitated with dry ether and washed 3 times, dissolved in water and lyophilized. Peptides were purified on a semi-preparative RP-C4 column (Vydac) with water containing 0.1% TFA and acetonitrile containing 0.08% TFA as mobile phase (yield: 40% (based on crude peptide); purity: >96%). The peptide mass was determined using a Voyager-DE Pro MALDI (Applied Biosystems) and a Thermo-FinniganLCQ ESI mass spectrometer.

### Example 2:

### Expression and Purification of Recombinant Glycosyltransferases

*Drosophila dGalNAcT1* and human *GalNAc-T2* were expressed as secreted N-terminally truncated proteins by *Baculovirus* infection of *T. ni* Hi-5 cells and semi-purified as previously described (Bennett et al., 1998; Schwientek et al., 2002). *Drosophila dC1GalT1* was expressed in a similar fashion by infection of Sf-9 cells with a *Baculovirus* expression construct encoding amino acids 48-388. The secreted N-terminally truncated protein was produced by virus amplification in Sf-9 cells and semi-purified from *T. ni* Hi-5 supernatants by consecutive size exclusion and ion-exchange chromatography on Q-Sepharose (pH 7.5). Recombinant *Drosophila dGlcAT-BSII* was expressed in *Drosophila* S2 cells as soluble fusion protein (residues 22-479) carrying a C-terminal V5- followed by a 6×His-tag. *dGlcAT-BSII* was purified by Immobilized Metal Ion Affinity Chromatography (IMAC) on Ni-NTA-beads as described by the manufacturer.

### Example 3:

### In-Vitro-Glycosylation of PEG₂₇-Peptide Conjugates

Quantitative conversion of the glycopeptides was achieved by highly efficient (near 100% completeness) enzymatic PEG₂₇-peptide conjugate glycosylation reactions. Effectivity of the glycosylation reactions was monitored by online-MS on a Bruker Ultaflextreme MALDI-TOF/TOF mass spectrometer.

### In vitro-Glycosylation of PA18 and PTA18 with the Tn-antigen

PA18 (H-PEG₂₇-AHGVTSAPDTRPAPGSTA-OH (SEQ ID NO:3)) and PTA18 (H-PEG₂₇-GDENLYFQAHGVTSAPDTRPAPGSTA-OH (SEQ ID NO:4)) were glycosylated *in vitro* using purified recombinant *Drosophila* or human glycosyltransferases. For the addition of the initial N-acetylgalactosamine, polypeptide *dGalNAcT1 (Drosophila)* or GalNAc-T2 (human) were used .[3;4] Tn-glycosylation of the peptide conjugates (1 mg peptide/mL) was performed in a reaction mixture containing 25 mM Tris-HCl buffer (pH 7.4), 10 mM MnC12, 0.1% Triton X-100, and 2 mM UDP-GalNAc at 37°C. Glycosylation of 1 mg PA18 and PTA18 with a single GalNAc per TR was accomplished by using *dGalNAcT1* (Figure 1). Incorporation of up to three moles of GalNAc was obtained with human GalNAc-T2 (data not shown).

### Product separation by micro GPC and diethyl ether/ethanol-precipitation

The glycosylated PA18 and PTA18 peptide conjugates were seperated from low molecular mass reaction components by gel permeation chromatography (GPC) on cross-linked dextran spin columns. Micro Bio-Spin columns (Bio-Rad) were prepacked with 0.5 ml of reconstituted Sephadex G-10 (GE LifeSciences) and washed with 5 column volumes of MQ water. The glycosylation reactions were applied to the GPC gel bed and a stacker volume of water was added to a total volume of 100 µl. The glycosylated peptide conjugates were recovered from the column by centrifugation (500 × g, 5 min) and half of the sample (50 µl each) was added to nine sample volumes of ethanol in Eppendorf tubes. The sample was mixed vigorously and the PEG₂₇-peptides then precipitated by the addition of two volumes (1 ml) of diethyl ether and incubation at -80°C for 4 h. The glycopeptide precipitates were harvested by centrifugation at 15,000 × g for 30 min, dried for 30 min at RT, and reconstituted in the appropriate buffer prior to the next glycosylation step.

### In vitro-Glycosylation of PA18-Tn and PTA18-Tn with the T-Antigen

PA18-Tn and PTA18-Tn were glycosylated *in vitro* using semi-purified recombinant *Drosophila dC1GalT1.* Glycosylation of the peptide conjugates (1 mg peptide/mL) with the T-antigen was performed in a reaction mixture containing 100 mM MES buffer (pH 6.5), 20 mM MnCl₂, 0.1% Triton X-100, 20 mM DTT and 2 mM UDP-Gal. Quantitative conversion of 1 mg PA18-Tn and PTA18-Tn to PA18-T and PTA18-T carrying a single T-Antigen in the MUC 1 TR was accomplished by overnight incubation at 37° C (Figures. 2 and 4).

### In vitro-Glycosylation of PA18-T and PTA18-T with the Glucuronyl-T-Antigen

PA18-T and PTA18-T were isolated by micro GPC and glycosylated *in vitro* using purified recombinant *Drosophila dGlcAT-BSII.* Glycosylation of the peptide conjugates (1 mg peptide/mL) with the glucuronyl T-antigen was performed in a reaction mixture containing 100 mM MES buffer (pH 6.5), 10 mM MnCl2, and 2 mM UDP-GlcA. Conversion of 1 mg PA18-T and PTA18-T to PA18-GT and PTA18-GT carrying a single glucuronyl T-antigen was generated by overnight incubation at 37°C (Figures. 2 and 4).

### Example 4:

### Enzymatic Release from the Polymer and Glycopeptide Product Purification

The glycosylated PTA18 conjugates were isolated from the final glycosylation reaction by micro GPC and diethyl ether/ethanol-precipitation. This was followed by digestion with Tobacco etch virus *(TEV)* protease resulting in the quantitative release of the final glycopeptide products from the PEGylated precursors. The reaction was performed in 50 mM Tris-HCl buffer (pH 8.0), 2 mM DTT, and 1 mM EDTA with a 1:20 ratio (w/w) of *TEV* protease to the PTA18 substrate for 3 h at RT. Completion of the reaction was monitored by MALDI-MS. The A18 glycopeptide products were separated from PEG₂₇-GDE8 and purified by reversed phase high-performance liquid chromatography (RP-HPLC) on a Vydac 218TP5415-C18 column (4.6 mm × 15 cm, 5 µm particle diameter, Grace Vydac) equipped with a 218GD54 guard (Grace Vydac) in a Beckman System Gold HPLC using 0.1% TFA and a gradient of 0-80% acetonitrile (Figures. 3 and 4). Quantification and estimation of yields of glycosylation reactions were performed by comparison of HPLC peaks by UV 210 absorbance using 10 µg weighed peptide as standard.

### References

1. Bennett EP, Hassan H, Mandel U, Mirgorodskaya E, Roepstorff P, Burchell J, Taylor-Papadimitriou J, Hollingsworth MA, Merkx G, van Kessel AG, Eiberg H, Steffensen R, and Clausen H; Cloning of a human UDP-N-acetyl-alpha-D-Galactosamine:polypeptide N-acetylgalactosaminyltransferase that complements other GalNAc-transferases in complete O-glycosylation of the MUC1 tandem repeat; J Biol Chem.; 1998; ;273(46):30472-30481.
2. Becker T, Dziadek S, Wittrock S, Kunz H; Synthetic glycopeptides from the mucin family as potential tools in cancer immunotherapy; Curr Cancer Drug Targets.; 2006; 6(6):491-517.
3. Carpino LA, and Han GY; 9-Fluorenylmethoxycarbonyl amino-protecting group; J. Org. Chem.; 1972; 37(22):3404―3409.
4. Merrifield RB; Solid Phase Synthesis I. The Synthesis of a Tetrapeptide; J. Am. Chem. Soc.; 1963; 85:2149-2154.
5. Schwientek T, Bennett EP, Flores C, Thacker J, Hollmann M, Reis CA, Behrens J, Mandel U, Keck B, Schäfer MA, Haselmann K, Zubarev R, Roepstorff P, Burchell JM, Taylor-Papadimitriou J, Hollingsworth MA, and Clausen H; Functional conservation of subfamilies of putative UDP-N-acetylgalactosamine:polypeptide N-acetylgalactosaminyltransferases in Drosophila, Caenorhabditis elegans, and mammals. One subfamily composed of 1(2)35Aa is essential in Drosophila; J Biol Chem.; 2002; 277(25):22623-22638.

## Claims

1. A method for the production of a glycosylated peptide comprising:
(i) providing a peptide conjugated with one or more hydrophilic polymer(s); and
(ii) glycosylating the polymer-conjugated peptide of step (i) by means of one or more glycosyltransferase(s).

2. The method of claim 1, further comprising the step of removing the glycosyltransferase(s) and/or reaction component(s) of step (ii).

3. The method of claim 2, wherein the step of removing the glycosyltransferase(s) and/or reaction component(s) is performed by chromatography and/or by precipitating the polymer-conjugated peptide, preferably by gel permeation chromatography (GPC) and/or precipitating the polymer-conjugated peptide, more preferably by a combination of GPC and precipitating the polymer-conjugated peptide, in particular by a combination of microspin GPC and precipitating the polymer-conjugated peptide.

4. The method of at least one of claims 1 to 3, wherein the peptide is a small peptide of less than 100 amino acids, preferably less than 50 amino acids, more preferably less than 40 amino acids, even more preferably less than 35 amino acids, more preferably less than 30 amino acids, even more preferably less than 25 amino acids, in particular less than 20 amino acids.

5. The method of at least one of claims 1 to 4, wherein the peptide is
(i) synthesized on a solid support, in particular by means of Fmoc- or Boc-based solid phase peptide synthesis (SPPS); and/or
(ii) conjugated with one or more hydrophilic polymer(s) on a solid support.

6. The method of claim 5, wherein the peptide is further cleaved from the solid support before glycosylation.

7. The method of at least one of claims 1 to 6, wherein conjugating the peptide with one or more hydrophilic polymer(s) is performed by site-specific conjugation, in particular by
(i) Native Chemical Ligation (NCL);
(ii) Staudinger Ligation, in particular Click Chemistry;
(iii) Maleimide-Thiol Conjugation;
(iv) Oxime formation;
(v) Thiazolidin Ligation;
(vi) Keto Acid-Hydroxy Amino (KAHA) Ligation; and/or
(vii) employing two or more different types of orthogonal protecting groups, in particular convergent solid phase peptide synthesis (SPPS).

8. The method of at least one of claims 1 to 7, wherein the one or more hydrophilic polymer(s) is/are selected from the group consisting of
(i) polyethylene glycol (PEG) or derivatives thereof;
(ii) polyethylene imine (PEI) or derivatives thereof;
(ii) polyacrylic acid or derivatives thereof, preferably methacrylic acid or derivative thereof, more preferably hydroxypropyl methacrylate (HPMA) or hydroxyethyl methacrylate (HEMA) or derivatives thereof, in particular N-(2-hydroxypropyl) methacrylamide (HPMA) or derivatives thereof;
(iv) polysaccharide(s) or derivatives thereof, preferably aminopolysaccharide(s), aminoalkyl polysaccharide(s), hydroxyalkyl polysaccharide(s) and/or alkyl polysaccharide(s);
(v) lipopolysaccharide(s)
(vi) hydrophilic polypeptide(s); and/or
(vii) conjugate(s) or blockpolymer(s) comprising two or more of the above,
preferably the hydrophilic polymer is PEG, more preferably a PEG polymer of between 5 to 50 PEG units, even more preferably of between 10 to 40 PEG units, even more preferably of between 15 to 35 PEG units, in particular of between 20 to 30 PEG units.

9. The method of at least one of claims 1 to 8, wherein the hydrophilic polymer is conjugated with the N- or C-terminus of the peptide, in particular the N-terminus of the peptide.

10. The method of at least one of claims 1 to 9, further comprising the step of cleaving the hydrophilic polymer(s) from the glycosylated peptide by cleaving at a cleavable site located between the glycosylation site(s) and the hydrophilic polymer(s).

11. The method of claim 10, wherein the cleavable site is
(i) an enzymatically cleavable site, preferably a proteolytic cleavage site, in particular a proteolytic cleavage site located in a sequence extension of the peptide;
(ii) a part of a photolabile linker; or
(iii) a part of a chemolabile linker.

12. The method of at least one of claims 1 to 11, further comprising the step of conjugating the peptide obtainable by a method of any one of claims 1 to 11 to another peptide.

13. The method of claim 12, wherein conjugating is performed by
(i) Native Chemical Ligation (NCL);
(ii) Click Chemistry;
(iii) Maleimide-Thiol Conjugation;
(iv) enzymatic conjugation, preferably a ligase- or a peptidase-based conjugation, in particular a subtiligase-, a trypsin- or a chymotrypsin-based conjugation;
(v) biochemical protein ligation, in particular expressed protein ligation (EPL) or protein trans-splicing (PTS);
(vi) soluble-handle conjugation, in particular Picolyl Ester Method; Thioester-Forming Ligation; Template-mediated Ligation; Capture-Mediated Ligation; and/or
(vii) employment of two or more different types of orthogonal protecting groups, in particular convergent solid phase peptide synthesis (SPPS).

14. The method of at least one of claims 1 to 13, further comprising the step of purifying the peptide, preferably by one or more chromatographic method(s) and/or one or more precipitation step(s), in particular gel permeation chromatography (GPC), fast protein liquid chromatography (FPLC), reversed phase high performance liquid chromatography (RP-HPLC) and/or diethyl ether precipitation.

15. The method of at least one of claims 1 to 14, comprising
(i) synthesizing a peptide on a solid support;
(ii) conjugating a hydrophilic polymer to the peptide of step (i) on the solid support;
(iii) cleaving the polymer-conjugated peptide of step (ii) off the solid support;
(iv) purifying the polymer-conjugated peptide of step (iii);
(v) glycosylating the peptide by means of a glycosyltransferase;
(vi) removing the one or more glycosyltransferase(s) and/or one or more reaction component(s) of step (v), preferably by means of chromatography, more preferably by means of GPC, in particular by means of microspin GPC; and
(vii) optionally reconstituting the peptide for the next glycosylation step and repeating the steps (v) and (vi).

16. The method of claim 15, further comprising the step of proteolytic cleavage in the peptide strand between the hydrophilic polymer and the glycosylation site.

17. The method of at least one of claims 1 to 16, further comprising:
(i) precipitating the peptide, in particular by means of diethyl ether precipitation;
(ii) drying the peptide, and/or
(iii) preserving the peptide, preferably by freeze-drying, freezing, addition of one or more preservative agent(s) and/or dissolution of the peptide in an organic solvent.
